# EUROPÄISCHE PATENTSCHRIFT

(11) **EP 2 618 755 B1**
(45) Veröffentlichungstag und Bekanntmachung des Hinweises auf die Patenterteilung: **19.11.2014**
(21) Anmeldenummer: 11758202.3
(22) Anmeldetag: 19.09.2011
(51) Int. Cl.: A61B 17/70, A61B 17/00

(54) **WIRBELSÄULENSTABILISIERUNGSSYSTEM UND CHIRURGISCHE VORRICHTUNG ZUM TEMPORÄREN VERSTEIFEN EINES FLEXIBLEN ZWISCHENABSCHNITTS EINES VERBINDUNGSELEMENTS DES WIRBELSÄULENSTABILISIERUNGSSYSTEMS**
SPINAL COLUMN STABILIZATION SYSTEM, AND SURGICAL DEVICE FOR TEMPORARILY STIFFENING A FLEXIBLE INTERMEDIATE PORTION OF A CONNECTING ELEMENT OF THE SPINAL COLUMN STABILIZATION SYSTEM
SYSTÈME DE STABILISATION DE LA COLONNE VERTÉBRALE ET DISPOSITIF CHIRURGICAL DE RAIDISSEMENT TEMPORAIRE D'UNE PARTIE INTERMÉDIAIRE SOUPLE D'UN ÉLÉMENT DE LIAISON DU SYSTÈME DE STABILISATION DE LA COLONNE VERTÉBRALE

(30) Priorität: 21.10.2010 DE 102010060101; 20.09.2010 DE 102010037666
(43) Veröffentlichungstag der Anmeldung: 31.07.2013
(73) Patentinhaber: Aesculap AG, 78532 Tuttlingen (DE)
(72) Erfinder: BEGER, Jens, 78532 Tuttlingen (DE); HOEFER, Fabian, 78532 Tuttlingen (DE); KRÜGER, Sven, 78647 Trossingen (DE)
(74) Vertreter: Hoeger, Stellrecht & Partner Patentanwälte
(86) Internationale Anmeldenummer: PCT/EP2011/066164
(87) Internationale Veröffentlichungsnummer: WO 2012/038354

(56) Entgegenhaltungen:
- WO-A1-2008/106303
- US-A1- 2007 276 380
- US-A1- 2008 234 747

## Beschreibung

Die vorliegende Erfindung betrifft eine chirurgische Vorrichtung zum temporären Versteifen eines mindestens teilweise flexiblen Zwischenabschnitts eines Verbindungselements für ein Wirbelsäulenstabilisierungssystem, welches Verbindungselement einen ersten Befestigungsabschnitt zum Festlegen an einer ersten Knochenbefestigungseinrichtung, einen zweiten Befestigungsabschnitt zum Festlegen an einer zweiten Knochenbefestigungseinrichtung und den zwischen dem ersten und dem zweiten Befestigungsabschnitt angeordneten oder ausgebildeten, mindestens teilweise flexiblen Zwischenabschnitt umfasst, welcher Zwischenabschnitt mindestens eine Ausnehmung definiert, die derart ausgebildet ist, dass sie eine Verformung des Zwischenabschnitts ermöglicht, wobei die chirurgische Vorrichtung mindestens ein Blockierelement umfasst, welches mindestens teilweise formschlüssig mit dem Zwischenabschnitt in Eingriff bringbar ist zum temporären Verhindern einer Verformung des flexiblen Zwischenabschnitts.

Ferner betrifft die vorliegende Erfindung ein Wirbelsäulenstabilisierungssystem umfassend mindestens eine erste Knochenbefestigungseinrichtung, mindestens eine zweite Knochenbefestigungseinrichtung und ein Verbindungselement, welches Verbindungselement einen ersten Befestigungsabschnitt zum Festlegen an einer ersten Knochenbefestigungseinrichtung, einen zweiten Befestigungsabschnitt zum Festlegen an einer zweiten Knochenbefestigungseinrichtung und einen zwischen dem ersten und dem zweiten Befestigungsabschnitt angeordneten oder ausgebildeten, mindestens teilweise flexiblen Zwischenabschnitt umfasst, welcher Zwischenabschnitt mindestens eine Ausnehmung definiert, die derart ausgebildet ist, dass sie eine Verformung des Zwischenabschnitts ermöglicht.

Ein Wirbelsäulenstabilisierungssystem der eingangs beschriebenen Art, insbesondere mit einem Verbindungselement, welches zwei Befestigungsabschnitte und einen dazwischen angeordneten flexiblen Zwischenabschnitt umfasst, ist beispielsweise aus der US 2006/0184171 A1 bekannt. Das Verbindungselement kann sich aufgrund des mindestens teilweise flexiblen Zwischenabschnitts beim Implantieren in unerwünschter Weise verformen. Insbesondere besteht diese Gefahr beim Einbringen und Verriegeln des Stabes an entsprechenden Knochenbefestigungseinrichtungen, beispielsweise an Knochenschrauben in Form von Pedikelschrauben.

Ein chirurgisches Instrument, welches zum temporären Versteifen des flexiblen Zwischenabschnitts verwendet werden kann ist beispielsweise aus der US 2008/0119862 A1 bekannt. Allerdings ist dieses Instrument nicht explizit für den Verwendungszweck vorgesehen und aufgrund seines Aufbaus für den praktischen Einsatz eher wenig geeignet.

Aus der US 2008/0234747 A1 ist ein resorbierbarer Lösemechanismus für ein chirurgisches Verbindungselement offenbart. In der US 2007/0276380 A1 ist eine Wirbelsäulenstabilisierungsvorrichtung beschrieben. Und schließlich sind aus der WO 2008/106303 A1 Instrumente und Verfahren zum minimalinvasiven Einsetzen dynamischer Platten bekannt.

Es ist daher Aufgabe der vorliegenden Erfindung, eine chirurgische Vorrichtung zum temporären Versteifen eines mindestens teilweise flexiblen Zwischenabschnitts eines Verbindungselements für ein Wirbelsäulenstabilisierungssystem sowie ein Wirbelsäulenstabilisierungssystem der eingangs beschriebenen Art so zu verbessern, dass die Implantation eines Verbindungselements mit einem mindestens teilweise flexiblen Zwischenabschnitt vereinfacht wird.

Diese Aufgabe wird bei einer chirurgischen Vorrichtung zum temporären Versteifen eines mindestens teilweise flexiblen Zwischenabschnitts eines Verbindungselements für ein Wirbelsäulenstabilisierungssystem gemäß der eingangs beschriebenen Art erfindungsgemäß dadurch gelöst, dass sie mindestens zwei in einer Bewegungsrichtung aufeinander zu und voneinander weg bewegbare umfasst und dass mindestens einer der Haltebacken das mindestens eine Blockierelement trägt oder umfasst.

Die erfindungsgemäß vorgeschlagene Lösung sieht somit vor, eine Verformung des Zwischenabschnitts durch direktes in Eingriff Bringen mit diesem zu verhindern. Zu diesem Zweck umfasst die chirurgische Vorrichtung mindestens ein Blockierelement. Dieses kann mindestens teilweise formschlüssig mit dem Zwischenabschnitt in Eingriff gebracht werden, um so temporär, beispielsweise beim Einbringen und Fixieren des Verbindungselements an entsprechenden Knochenbefestigungseinrichtungen, eine unerwünschte Verformung des flexiblen Zwischenabschnitts zu verhindern. Gleichzeitig kann, und zwar abhängig von dessen Ausgestaltung, das mindestens eine Blockierelement auch den Zwischenabschnitt schützen, um eine Beschädigung desselben, insbesondere eine unerwünschte Verformung, zu verhindern. Die chirurgische Vorrichtung kann insbesondere auf vielfältige Weise ausgebildet sein. Bevorzugte Ausführungsformen werden nachfolgend beschrieben sowie in den Unteransprüchen näher definiert. Die chirurgische Vorrichtung kann bei entsprechender Ausgestaltung insbesondere das Verbindungselement in allen Bewegungsrichtungen vorzugsweise für den Zeitraum der Implantation, also während des Einbringens des Verbindungselements und während des Verriegelns desselben an den Knochenbefestigungseinrichtungen, temporär versteifen, also eine Verformung desselben verhindern. Optional kann die chirurgische Vorrichtung nach dem Verriegeln der Knochenbefestigungseinrichtungen, also nach dem endgültigen Festlegen des Verbindungselements an denselben, entfernt werden. Die Haltebacken mit ihren Halteflächen können auf einfache Weise eine Bewegung des Zwischenabschnitts des Verbindungselements in einer Richtung quer zu den Halteflächen verhindern. Damit können je nach Ausgestaltung der Haltebacken ein Freiheitsgrad oder sogar alle Bewegungsfreiheitsgrade des Zwischenabschnitts jeweils ganz oder teilweise eingeschränkt werden. Besonders einfach und kompakt kann der Aufbau der chirurgischen Vorrichtung erreicht werden, dass mindestens einer der Haltebacken das mindestens eine Blockierelement trägt oder umfasst. Die Vorrichtung kann insbesondere so ausgebildet sein, dass nur einer der Haltebacken ein oder mehrere Blockierelemente umfasst. Es können aber auch zwei oder mehr Haltebacken jeweils ein oder mehrere Blockierelemente tragen, die dann zum temporären Verhindern einer Bewegung des Zwischenabschnitts zusammenwirken.

Günstig kann es ferner sein, wenn die mindestens zwei Haltebacken insgesamt mindestens zwei aufeinander zu weisende Axialanschläge umfassen, welche in einer Richtung quer zur Bewegungsrichtung der Haltebacken relativ zueinander wirkend ausgebildet sind zum Verhindern einer Bewegung des Zwischenabschnitts in axialer Richtung. Unter einer Bewegung des Zwischenabschnitts in axialer Richtung ist insbesondere eine Verformung, das heißt eine Kompression oder Dehnung des Zwischenabschnitts, in axialer Richtung zu verstehen. Die vorgeschlagenen Axialanschläge können insbesondere eine Dehnung des Zwischenabschnitts verhindern und somit dazu dienen, einen Bewegungsfreiheitsgrad des Zwischenabschnitts einzuschränken.

Um sicherstellen zu können, dass die chirurgische Vorrichtung ein Verbindungselement mit blockiertem Zwischenabschnitt nicht unabsichtlich freigibt, ist es vorteilhaft, wenn die chirurgische Vorrichtung eine Verriegelungseinrichtung zum Verhindern einer Bewegung der Haltebacken in der Bewegungsrichtung voneinander weg in einer Blockierstellung umfasst. Mit der Verriegelungseinrichtung kann also die chirurgische Vorrichtung vorzugsweise temporär in der Blockierstellung gehalten werden.

Besonders einfach und kompakt kann der Aufbau der chirurgischen Vorrichtung erreicht werden, wenn mindestens einer der Haltebacken das mindestens eine Blockierelement trägt oder umfasst. Die Vorrichtung kann insbesondere so ausgebildet sein, dass nur einer der Haltebacken ein oder mehrere Blockierelemente umfasst. Es können aber auch zwei oder mehr Haltebacken jeweils ein oder mehrere Blockierelemente tragen, die dann zum temporären Verhindern einer Bewegung des Zwischenabschnitts zusammenwirken.

Auf besonders einfache Weise lässt sich der mindestens teilweise flexible Zwischenabschnitt gegen eine Verformung sichern, wenn das mindestens eine Blockierelement in Form eines von einer Haltefläche eines der Haltebacken abstehenden Blockiervorsprungs ausgebildet ist. Zudem lässt sich eine solche chirurgische Vorrichtung auf einfache Weise herstellen.

Vorteilhafterweise kann der Blockiervorsprung keilförmig oder im Wesentlichen keilförmig ausgebildet sein. Dies ist insbesondere dann vorteilhaft, wenn Ausnehmungen des Zwischenabschnitts korrespondierend ausgebildet sind. So lassen sich auf einfache Weise gezielt Bewegungsfreiheitsgrade des Zwischenabschnitts in mehr als einer Raumrichtung blockieren.

Besonders günstig ist es, wenn das mindestens eine Blockierelement in Form einer an einem der Haltebacken in der Haltefläche vorgesehenen Blockierausnehmung zum mindestens teilweisen Aufnehmen des temporär zu versteifenden Zwischenabschnitts ausgebildet ist. Insbesondere ist es so auch möglich, den Zwischenabschnitt in einer oder zwei Blockierausnehmungen der Haltebacken ganz oder teilweise aufzunehmen. Insbesondere kann auf diese Weise der Zwischenabschnitt während der Implantation besonders gut geschützt werden.

Der Aufbau der chirurgischen Vorrichtung lässt sich weiter vereinfachen, wenn mindestens einer der Axialanschläge an einer Blockierausnehmung ausgebildet ist. Insbesondere können auch zwei aufeinander zu weisende Axialanschläge an einer Blockierausnehmung ausgebildet sein. Die beiden Haltebacken mit mindestens einer, gegebenenfalls auch zwei Blockierausnehmungen können dann zusammenwirken und optional sogar sämtliche Bewegungsfreiheitsgrade des Zwischenabschnitts blockieren.

Je nach Ausgestaltung des Zwischenabschnitts kann es vorteilhaft sein, wenn die mindestens zwei Haltebacken unterschiedlich ausgebildet sind. Dies bedeutet insbesondere, dass sie unterschiedlich geformt sein und auch eine unterschiedliche Zahl an Blockierelementen tragen oder umfassen können. Insbesondere können auch die Ausgestaltungen der Blockierelemente voneinander abweichen. Beispielsweise kann ein Haltebacken eine Blockierausnehmung aufweisen, ein anderer Haltebacken einen Blockiervorsprung.

Günstig ist es, wenn die Haltebacken unterschiedlich geformte Blockierelemente umfassen. Dies gestattet es, die Haltebacken individuell an das Verbindungselement, das heißt insbesondere an dessen Zwischenabschnitt, anzupassen, um eine Verformung desselben insbesondere während der Implantation zu verhindern. Insbesondere kann bei unterschiedlich geformten Blockierelementen die chirurgische Vorrichtung insgesamt besonders kompakt ausgebildet werden.

Gemäß einer weiteren bevorzugten Ausführungsform der Erfindung kann die chirurgische Vorrichtung eine Kupplungseinrichtung zum lösbaren Verbinden der zwei Haltebacken miteinander in einer Kupplungsstellung vorsehen. Dies gestattet es insbesondere, die chirurgische Vorrichtung ausschließlich durch zwei miteinander verbindbare Haltebacken auszubilden, die vor der Implantation mit dem Verbindungselement in Eingriff gebracht und mittels der Kupplungseinrichtung in der Kupplungsstellung gehalten werden können. Nach Festlegen des Verbindungselements an entsprechenden Knochenbefestigungseinrichtungen kann dann die chirurgische Vorrichtung wieder entfernt werden, und zwar durch Lösen der Kupplungseinrichtung und Entfernen der Haltebacken vom Zwischenabschnitt. Dieser kann sich dann in gewünschter Weise unter Krafteinwirkung im Körper des Patienten verformen.

Besonders einfach wird der Aufbau der Kupplungseinrichtung, wenn diese erste und zweite Kupplungselemente umfasst, welche einerseits am einen Haltebacken und andererseits am anderen Haltebacken angeordnet oder ausgebildet sind und in der Kupplungsstellung in Eingriff stehen. Die Kupplungselemente können beispielsweise in Form von Rastelementen ausgebildet sein. Denkbar sind auch miteinander verschraubbare Elemente, die zueinander korrespondierende Innen- und Außengewinde tragen können.

Besonders einfach lassen sich zwei Haltebacken miteinander verbinden und in einer Kupplungsstellung halten, wenn die Kupplungseinrichtung einen Kupplungssteg oder einen Faden umfasst. Insbesondere können die Haltebacken auch mittels eines Fadens zusammengehalten werden. Ein an einem Haltebacken abstehender Kupplungssteg kann beispielsweise eine korrespondierende Ausnehmung am anderen Haltebacken durchsetzen und mit dieser eine rastende Verbindung in der Kupplungsstellung eingehen.

Die oben beschriebenen bevorzugten Ausführungsformen chirurgischer Vorrichtungen zum temporären Versteifen eines mindestens teilweise flexiblen Zwischenabschnitts eines Verbindungselements können insbesondere derart ausgebildet sein, dass sie direkt am Zwischenabschnitt angreifen, klein und kompakt ausgebildet sind sowie nach dem Implantieren des Verbindungselements wieder von diesem entfernt werden können. Zur Verbesserung einer Handhabung der chirurgischen Vorrichtung kann diese vorteilhafterweise eine Betätigungseinrichtung zum Überführen der Vorrichtung von einer Anlagestellung, in welcher die Vorrichtung und der Zwischenabschnitt außer Eingriff stehen, in eine Blockierstellung umfassen, in welcher mit der Vorrichtung der Zwischenabschnitt unverformbar an der Vorrichtung gehalten ist. Die Betätigungseinrichtung ermöglicht es zum Beispiel auf einfache Weise, die Vorrichtung an den Zwischenabschnitt anzulegen und gegebenenfalls von diesem zu entfernen. Die Betätigungseinrichtung ermöglicht somit eine besonders einfache Handhabung der chirurgischen Vorrichtung.

Die Handhabung der chirurgischen Vorrichtung kann insbesondere dadurch weiter verbessert werden, dass die Betätigungseinrichtung zwei relativ zueinander bewegbare Betätigungsglieder umfasst, welche direkt oder indirekt mit den mindestens zwei Haltebacken gekoppelt sind zum Bewegen derselben. Mit anderen Worten können die Haltebacken auch direkt mit der Betätigungseinrichtung verbunden sein, so dass insbesondere ein chirurgisches Instrument ausgebildet wird, welches die chirurgische Vorrichtung zum temporären Versteifen eines mindestens teilweise flexiblen Zwischenabschnitts eines Verbindungselements für ein Wirbelsäulenstabilisierungssystem umfasst. Eine solche Ausbildung ermöglicht es einem Operateur, die chirurgische Vorrichtung nicht nur zum Blockieren des Zwischenabschnitts zu nutzen, sondern auch zum Halten und Einsetzen des Verbindungselements insgesamt während der Implantation desselben. Ein solches chirurgisches Instrument kann also gleichzeitig zwei Funktionen ausüben. Zum einen kann es den Zwischenabschnitt temporär versteifen und zum anderen als Halteinstrument für das Verbindungselement genutzt werden. Dies ist insbesondere bei minimalinvasiven chirurgischen Eingriffen vorteilhaft, da in diesen Fällen nur ein begrenzter räumlicher Zugang zum Operationssitus möglich ist. Selbstverständlich kann das Instrument auch zum perkutanen Einsetzen der chirurgischen Vorrichtung durch die Muskulatur verwendet werden.

Die eingangs gestellte Aufgabe wird ferner bei einem Wirbelsäulenstabilisierungssystem der eingangs beschriebenen Art erfindungsgemäß dadurch gelöst, dass es eine der oben beschriebenen chirurgischen Vorrichtungen zum temporären Versteifen des flexiblen Zwischenabschnitts des Verbindungselements umfasst.

Ein solches Wirbelsäulenstabilisierungssystem lässt sich aufgrund der besonderen Ausgestaltung des Verbindungselements auf besonders einfache Weise und abhängig von der konkreten Ausgestaltung der chirurgischen Vorrichtung zum temporären Versteifen des flexiblen Zwischenabschnitts des Verbindungselements auch besonders schonend für den Patienten implantieren. Es weist im Übrigen die eingangs beschriebenen Vorteile der vorgeschlagenen, verbesserten chirurgischen Vorrichtung auf.

Vorteilhaft ist es, wenn der Zwischenabschnitt in Form eines streifenförmigen, gewundenen Blattfederelements ausgebildet ist und mindestens eine in einer Richtung quer zu einer vom Zwischenabschnitt definierten Längsachse seitlich geöffnete Ausnehmung aufweist. Ein solcher blattfederförmiger Zwischenabschnitt des Verbindungselements ist auf einfache Weise herzustellen, beispielsweise aus einem streifenförmigen Material durch Biegen oder aus einem Vollmaterial durch spanende Bearbeitung, zum Beispiel durch Fräsen. Ferner können die Ausnehmungen günstigerweise dazu dienen, Blockierelemente der chirurgischen Vorrichtung aufzunehmen, um den Zwischenabschnitt temporär zu versteifen.

Bei den weiteren Ausführungsbeispielen handelt es sich nicht um einen Teil der Erfindung. Sie dienen dem Verständnis der Erfindung.

Vorteilhaft ist es, wenn das mindestens eine Blockierelement mindestens teilweise in die mindestens eine Ausnehmung einführbar ist zum temporären Verhindern einer Verformung des flexiblen Zwischenabschnitts. Ein in eine Ausnehmung des Zwischenabschnitts eingeführtes Blockierelement kann insbesondere einen Abschnitt des Zwischenabschnitts gegen Verformung blockieren.

Eine optimale Wirksamkeit der Vorrichtung kann insbesondere dadurch erreicht werden, dass das mindestens eine Blockierelement korrespondierend oder im Wesentlichen korrespondierend zu einer Ausnehmung des Zwischenäbschnitts ausgebildet ist. Beispielsweise lassen sich so Bewegungen in Richtung einer vom Verbindungselement definierten Längsachse oder optional auch entsprechende Schwenkbewegungen um Schwenkachsen quer zur Längsachse auf einfache Weise verhindern. Ferner kann durch die korrespondierende Ausbildung des mindestens einen Blockierelements auch ein optimaler temporärer Schutz des Zwischenabschnitts erreicht werden.

Besonders einfach lässt sich eine Verformung des Zwischenabschnitts temporär verhindern, wenn das mindestens eine Blockierelement formschlüssig oder im Wesentlichen formschlüssig in eine Ausnehmung des Zwischenabschnitts einführbar ist. Das mindestens eine Blockierelement kann so auf einfache Weise in die Ausnehmung des Zwischenabschnitts eingeführt werden, zum Beispiel für die Zeitdauer der Implantation, und nach abgeschlossener Implantation wieder aus der Ausnehmung entfernt werden, beispielsweise durch Herausziehen.

Die Konstruktion und Herstellung der chirurgischen Vorrichtung lässt sich weiter vereinfachen, wenn der Blockiervorsprung in Form eines zylindrischen Zapfens ausgebildet ist.

Der Aufbau der chirurgischen Vorrichtung wird besonders einfach, wenn die Betätigungsglieder verschwenkbar aneinander gelagert sind. Auf diese Weise lässt sich die chirurgische Vorrichtung beispielsweise in Form einer Klemm- oder Fasszange ausbilden, wobei die Haltebacken distale Enden der Zange in Form von Werkzeugelementen bilden, mit denen der Zwischenabschnitt gefasst und gehalten werden kann.

Vorzugsweise bildet die chirurgische Vorrichtung einen Teil eines chirurgischen Klemm- oder Halteinstruments. Wie bereits beschrieben können beispielsweise die Haltebacken der chirurgischen Vorrichtung Werkzeugelemente einer chirurgischen Fass- oder Klemmzange bilden. Das Klemm- oder Halteinstrument kann insbesondere auch als endoskopisches Rohrschaftinstrument mit einem langgestreckten rohrförmigen Schaft ausgebildet sein, um insbesondere den Einsatz bei minimalinvasiven chirurgischen Eingriffen zu ermöglichen.

Gemäß einer weiteren bevorzugten Ausführungsform der Erfindung kann die chirurgische Vorrichtung einstückig ausgebildet sein und den flexiblen Zwischenabschnitt formschlüssig umgeben. Sie kann ihn auch nur teilweise formschlüssig umgeben, jedoch derart, dass eine Verformung des Zwischenabschnitts ohne Entfernen der chirurgischen Vorrichtung nicht möglich ist, also derart, dass vorzugsweise alle Bewegungsfreiheitgrade des Zwischenabschnitts blockiert sind. Einstückig ausgebildet bedeutet insbesondere auch, dass die chirurgische Vorrichtung den Zwischenabschnitt mindestens an einer Stellung ringförmig umschließt oder mindestens teilweise ringförmig umschließen kann, um ihn in einer Implantationsstellung unverformbar zu fixieren. Die chirurgische Vorrichtung kann in diesem Fall bereits nach der Herstellung des Verbindungselements am Zwischenabschnitt festgelegt werden und steht somit einem Operateur bereits als Teil des Verbindungselements zur Verfügung. Nach erfolgreicher Implantation des Verbindungselements kann dann die chirurgische Vorrichtung wieder vom Zwischenabschnitt entfernt werden.

Besonders einfach und kostengünstig herstellen lässt sich die chirurgische Vorrichtung, wenn sie mindestens teilweise aus einem flüssigkeitslöslichen Material hergestellt ist. Dies gestattet es, nach Herstellung des Verbindungselements den Zwischenabschnitt ganz oder teilweise in das flüssigkeitslösliche Material einzubetten, welches in Abwesenheit einer Flüssigkeit, beispielsweise Wasser, eine ausreichende Festigkeit aufweist, um ein Verformen des flexiblen Zwischenabschnitts zu verhindern.

Besonders kostengünstig und körperverträglich herstellen lässt sich die chirurgische Vorrichtung, wenn das flüssigkeitslösliche Material Zucker oder Salz ist oder wenn es Zucker oder Salz enthält. Die chirurgische Vorrichtung kann dann auf einfache Weise durch Spülen beispielsweise mit Wasser vom Verbindungselement abgelöst werden, um den Zwischenabschnitt freizugeben.

Gemäß einer weiteren bevorzugten Ausführungsform der Erfindung kann vorgesehen sein, dass die chirurgische Vorrichtung mindestens teilweise aus einem Material hergestellt ist, dessen Aggregatzustand zum Entfernen der Vorrichtung änderbar ist. Vorzugsweise ist das Material von einem festen in einen flüssigen oder gasförmigen Zustand überführbar, beispielsweise durch Änderung von Umgebungsbedingungen wie zum Beispiel Temperatur oder Druck. Beispielsweise kann das Material Wasser in Form von Eis oder ein Kunststoff sein.

Günstig ist es, wenn die chirurgische Vorrichtung mindestens teilweise aus einem Material hergestellt ist, welches in Folge einer Temperaturänderung seine Festigkeit dauerhaft ändert. Beispielsweise kann das Verbindungselement mit der chirurgischen Vorrichtung versehen und bei einer tiefen Temperatur implantiert werden. Wird die Temperatur zumindest im Bereich der chirurgischen Vorrichtung erhöht, kann diese ihre Festigkeit teilweise oder ganz verlieren, um so dauerhaft eine Verformung des Zwischenabschnitts zu ermöglichen.

Besonders einfach herstellbar ist die chirurgische Vorrichtung, wenn das Material Eis oder ein biokompatibler Kunststoff ist. Beispielsweise lässt sich der Zwischenabschnitt ganz oder teilweise in einen Eismantel einhüllen, um den Zwischenabschnitt zu versteifen. In Frage kommen auch biokompatible Kunststoffe, die bei Erwärmung über ihre Fließtemperatur aufweichen, dabei ihre ursprüngliche Steifigkeit verlieren und so ein Verformen des Zwischenabschnitts in gewünschter Weise ermöglichen.

Besonders vorteilhaft ist es, wenn der Kunststoff resorbierbar ist. Dies gestattet es insbesondere, zunächst ein steifes, das heißt unflexibles Verbindungselement einzusetzen, dessen Zwischenabschnitt mittels der chirurgischen Vorrichtung versteift wird. Nach Implantation kann durch Resorption des Kunststoffes die chirurgische Vorrichtung auf einfache Weise vom Körper des Patienten selbstständig entfernt werden.

Der Aufbau der chirurgischen Vorrichtung wird besonders einfach, wenn die Betätigungsglieder verschwenkbar aneinander gelagert sind. Auf diese Weise lässt sich die chirurgische Vorrichtung beispielsweise in Form einer Klemm- oder Fasszange ausbilden, wobei die Haltebacken distale Enden der Zange in Form von Werkzeugelementen bilden, mit denen der Zwischenabschnitt gefasst und gehalten werden kann.

Vorzugsweise bildet die chirurgische Vorrichtung einen Teil eines chirurgischen Klemm- oder Halteinstruments. Wie bereits beschrieben können beispielsweise die Haltebacken der chirurgischen Vorrichtung Werkzeugelemente einer chirurgischen Fass- oder Klemmzange bilden. Das Klemm- oder Halteinstrument kann insbesondere auch als endoskopisches Rohrschaftinstrument mit einem langgestreckten rohrförmigen Schaft ausgebildet sein, um insbesondere den Einsatz bei minimalinvasiven chirurgischen Eingriffen zu ermöglichen.

Gemäß einer weiteren bevorzugten Ausführungsform der Erfindung kann die chirurgische Vorrichtung einstückig ausgebildet sein und den flexiblen Zwischenabschnitt formschlüssig umgeben. Sie kann ihn auch nur teilweise formschlüssig umgeben, jedoch derart, dass eine Verformung des Zwischenabschnitts ohne Entfernen der chirurgischen Vorrichtung nicht möglich ist, also derart, dass vorzugsweise alle Bewegungsfreiheitgrade des Zwischenabschnitts blockiert sind. Einstückig ausgebildet bedeutet insbesondere auch, dass die chirurgische Vorrichtung den Zwischenabschnitt mindestens an einer Stellung ringförmig umschließt oder mindestens teilweise ringförmig umschließen kann, um ihn in einer Implantationsstellung unverformbar zu fixieren. Die chirurgische Vorrichtung kann in diesem Fall bereits nach der Herstellung des Verbindungselements am Zwischenabschnitt festgelegt werden und steht somit einem Operateur bereits als Teil des Verbindungselements zur Verfügung. Nach erfolgreicher Implantation des Verbindungselements kann dann die chirurgische Vorrichtung wieder vom Zwischenabschnitt entfernt werden.

Besonders einfach und kostengünstig herstellen lässt sich die chirurgische Vorrichtung, wenn sie mindestens teilweise aus einem flüssigkeitslöslichen Material hergestellt ist. Dies gestattet es, nach Herstellung des Verbindungselements den Zwischenabschnitt ganz oder teilweise in das flüssigkeitslösliche Material einzubetten, welches in Abwesenheit einer Flüssigkeit, beispielsweise Wasser, eine ausreichende Festigkeit aufweist, um ein Verformen des flexiblen Zwischenabschnitts zu verhindern.

Besonders kostengünstig und körperverträglich herstellen lässt sich die chirurgische Vorrichtung, wenn das flüssigkeitslösliche Material Zucker oder Salz ist oder wenn es Zucker oder Salz enthält. Die chirurgische Vorrichtung kann dann auf einfache Weise durch Spülen beispielsweise mit Wasser vom Verbindungselement abgelöst werden, um den Zwischenabschnitt freizugeben.

Gemäß einer weiteren bevorzugten Ausführungsform der Erfindung kann vorgesehen sein, dass die chirurgische Vorrichtung mindestens teilweise aus einem Material hergestellt ist, dessen Aggregatzustand zum Entfernen der Vorrichtung änderbar ist. Vorzugsweise ist das Material von einem festen in einen flüssigen oder gasförmigen Zustand überführbar, beispielsweise durch Änderung von Umgebungsbedingungen wie zum Beispiel Temperatur oder Druck. Beispielsweise kann das Material Wasser in Form von Eis oder ein Kunststoff sein.

Günstig ist es, wenn die chirurgische Vorrichtung mindestens teilweise aus einem Material hergestellt ist, welches in Folge einer Temperaturänderung seine Festigkeit dauerhaft ändert. Beispielsweise kann das Verbindungselement mit der chirurgischen Vorrichtung versehen und bei einer tiefen Temperatur implantiert werden. Wird die Temperatur zumindest im Bereich der chirurgischen Vorrichtung erhöht, kann diese ihre Festigkeit teilweise oder ganz verlieren, um so dauerhaft eine Verformung des Zwischenabschnitts zu ermöglichen.

Besonders einfach herstellbar ist die chirurgische Vorrichtung, wenn das Material Eis oder ein biokompatibler Kunststoff ist. Beispielsweise lässt sich der Zwischenabschnitt ganz oder teilweise in einen Eismantel einhüllen, um den Zwischenabschnitt zu versteifen. In Frage kommen auch biokompatible Kunststoffe, die bei Erwärmung über ihre Fließtemperatur aufweichen, dabei ihre ursprüngliche Steifigkeit verlieren und so ein Verformen des Zwischenabschnitts in gewünschter Weise ermöglichen.

Besonders vorteilhaft ist es, wenn der Kunststoff resorbierbar ist. Dies gestattet es insbesondere, zunächst ein steifes, das heißt unflexibles Verbindungselement einzusetzen, dessen Zwischenabschnitt mittels der chirurgischen Vorrichtung versteift wird. Nach Implantation kann durch Resorption des Kunststoffes die chirurgische Vorrichtung auf einfache Weise vom Körper des Patienten selbstständig entfernt werden.

Vorteilhaft ist es, wenn das Verbindungselement aus einem metallischen Material oder einem Kunststoff hergestellt ist. Je nach erforderlicher Steifigkeit kann das eine oder das andere Material zur Herstellung des Verbindungselements gewählt werden. Insbesondere können durch Wahl des Materials praktisch beliebige Steifigkeiten eingestellt werden. Vorzugsweise liegen diese in einem Bereich von etwa 30 N/mm bis 150 N/mm.

Vorzugsweise ist das metallische Material Titan, eine Titanlegierung oder eine Kobalt-Chrom-Legierung oder enthält die genannten Materialien. Insbesondere handelt es sich dabei um körperverträgliche metallische Materialien.

Günstigerweise ist der Kunststoff Polyetheretherketon (PEEK) oder Kohlefaser verstärktes Polyetheretherketon (PEEK) oder enthält die genannten Materialien. Insbesondere die genannten Materialien zeichnen sich durch eine hohe Körperverträglichkeit aus.

Besonders einfach herstellen lässt sich das Verbindungselement des Wirbelsäulenstabilisierungssystems, wenn es spiegelsymmetrisch oder im Wesentlichen spiegelsymmetrisch zu einer Spiegelebene ausgebildet ist. Im Wesentlichen spiegelsymmetrische bedeutet insbesondere, dass aufgrund von Fertigungstoleranzen bei einer Herstellung des Zwischenabschnitts aus einem Vollmaterial keine durchgehende Blattfederelementoberfläche hergestellt werden, sondern dass diese mindestens einen einstufigen Absatz aufgrund leicht versetzt eingeführter Fräser aufweisen kann.

Die Stabilität sowie die Biegeeigenschaften des Verbindungselements lassen sich besonders einfach und hochpräzise einstellen, wenn die Spiegelebene eine vom Verbindungselement definierte Längsachse enthält.

Das Verbindungselement lässt sich insbesondere hervorragend als Ersatz eines geraden, stabförmigen Verbindungselements nutzen, wenn die Längsachse Längsachsen der Befestigungsabschnitte definiert.

Die nachfolgende Beschreibung bevorzugter Ausführungsformen der Erfindung dient im Zusammenhang mit der Zeichnung der näheren Erläuterung. Die Figuren 12-15 zeigen jedoch keine erfindungsgemäßen Verbindungselemente und die Figur 21 zeigt keine erfindungsgemäße chirurgische Vorrichtung. Es zeigen:
- Figur 1:: eine schematische Gesamtansicht eines an einer Wirbelsäule festgelegten Wirbelsäulenstabilisierungssystems;
- Figur 2:: eine schematische perspektivische Gesamtansicht eines chirurgischen Instruments umfassend eine chirurgische Vorrichtung zum temporären Versteifen eines in Figur 1 dargestellten, mindestens teilweisen flexiblen Zwischenabschnitts eines Verbindungselements des Wirbelsäulenstabilisierungssystems;
- Figur 3:: eine perspektivische Ansicht eines distalen Endes des in Figur 2 dargestellten chirurgischen Instruments vor dem Fassen oder nach dem Lösen des Zwischenabschnitts des Verbindungselements;
- Figur 3A:: eine vergrößerte Ansicht des Bereichs A in Figur 3;
- Figur 4:: eine Ansicht ähnlich Figur 3 des chirurgischen Instruments aus Figur 3 von der Gegenseite;
- Figur 5:: eine schematische Darstellung eines Haltebackens der chirurgischen Vorrichtung aus den Figuren 2 und 3 im Eingriff mit dem Zwischenabschnitt;
- Figur 6:: eine Ansicht analog Figur 4 einer alternativen Ausführungsform eines Haltebackens;
- Figur 7:: eine schematische Gesamtansicht des Verbindungselements mit den in den Figuren 5 und 6 dargestellten Haltebacken zur Ausbitdung einer chirurgischen Vorrichtung zum Versteifen des Zwischenabschnitts;
- Figur 8:: eine schematische Seitenansicht einer weiteren Ausführungsform eines Haltebackens der chirurgischen Vorrichtung in einer Seitenansicht im Eingriff mit dem Zwischenabschnitt des Verbindungselements;
- Figur 9:: eine perspektivische Ansicht einer weiteren Ausführungsform eines Haltebackens vor dem in Eingriff Bringen mit dem Zwischenabschnitt des Verbindungselements;
- Figur 10:: eine Seitenansicht des Haltebackens aus Figur 9 im Eingriff mit dem Zwischenabschnitt;
- Figur 11:: eine Schnittansicht längs Linie 11-11 in Figur 10;
- Figur 12:: eine schematische perspektivische Ansicht einer weiteren Ausführungsform eines Haltebackens vor dem Anlegen an ein nicht erfindungsgemäßes Verbindungselement mit einem flexiblen Zwischenabschnitt;
- Figur 13:: eine schematische perspektivische Ansicht des Haltebackens aus Figur 12 im Eingriff mit dem flexiblen Zwischenabschnitt;
- Figur 14:: eine teilweise durchbrochene Seitenansicht der in Figur 13 dargestellten Anordnung;
- Figur 15:: eine schematische perspektivische Ansicht einer weiteren Ausführungsform eines Haltebackens vor dem Anlegen an ein nicht erfindungsgemäßes Verbindungselement mit einem flexiblen Zwischenabschnitt;
- Figur 16:: eine schematische perspektivische Ansicht einer weiteren Ausführungsform eines Haltebackens vor dem Anlegen an ein Verbindungselement mit einem flexiblen Zwischenabschnitt;
- Figur 17:: eine schematische perspektivische Ansicht des Haltebackens aus Figur 16 im Eingriff mit dem flexiblen Zwischenabschnitt;
- Figur 18:: eine schematische perspektivische Ansicht einer chirurgischen Vorrichtung umfassend zwei mittels einer Kupplungseinrichtung gekoppelter Haltebacken;
- Figur 19:: eine Seitenansicht der in Figur 18 dargestellten chirurgischen Vorrichtung;
- Figur 20:: eine Seitenansicht ähnlich Figur 19 eines weiteren Ausführungsbeispiels einer chirurgischen Vorrichtung; und
- Figur 21:: eine perspektivische Ansicht eines weiteren Ausführungsbeispiels einer chirurgischen Vorrichtung im Eingriff mit einem flexiblen Zwischenabschnitt eines Verbindungselements.

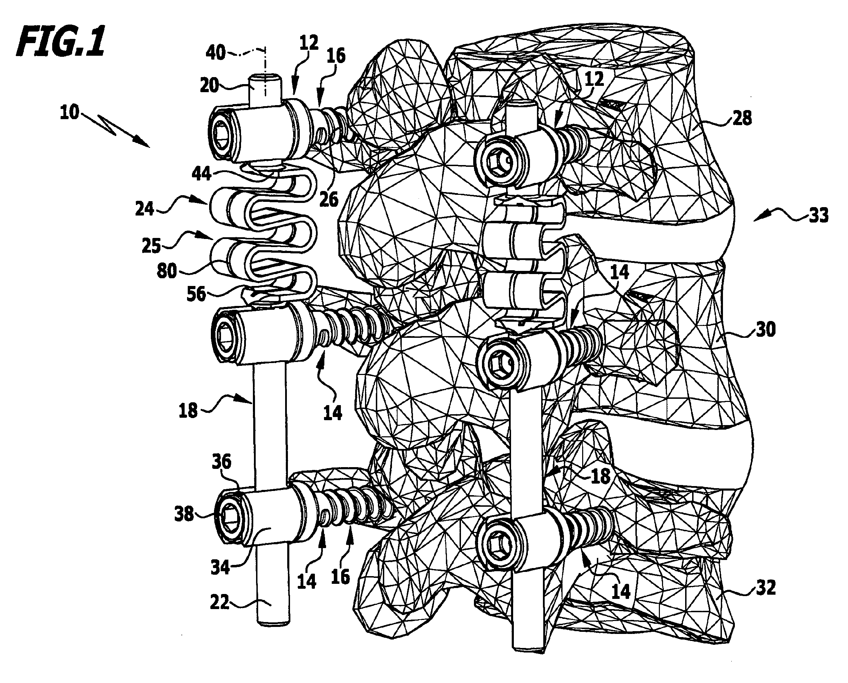

In Figur 1 ist schematisch ein insgesamt mit dem Bezugszeichen 10 bezeichnetes Wirbelsäulenstabilisierungssystem dargestellt. Es umfasst erste Knochenbefestigungseinrichtungen 12 und zweite Knochenbefestigungseinrichtungen 14, die bei dem in Figur 1 schematisch dargestellten Ausführungsbeispiel alle in Form identischer Knochenschrauben 16 ausgebildet sind, jedoch auch unterschiedlich ausgebildet sein können. Ferner umfasst das Wirbelsäulenstabilisierungssystem 10 im Wesentlichen stabförmige Verbindungselemente 18, welche einen ersten Befestigungsabschnitt 20 zum Festlegen an einer Knochenschraube 16, einen zweiten Befestigungsabschnitt 22 zum Festlegen an zwei Knochenschrauben 16 und einen zwischen dem ersten und dem zweiten Befestigungsabschnitt 20, 22 angeordneten oder ausgebildeten, mindestens teilweise flexiblen Zwischenabschnitt 24 in Form eines Blattfederelements 25 umfassen.

Die Knochenschrauben 16 umfassen jeweils einen Knochenverankerungsabschnitt 26 mit einem Knochengewinde zum Verankern in einem Knochen, beispielsweise in einem der in Figur 1 schematisch dargestellten Wirbel 28, 30 und 32 einer Wirbelsäule 33. Des Weiteren umfasst jede Knochenschraube 16 einen in einer Justagestellung gelenkig am Knochenverankerungsabschnitt 26 gelagerten Halteabschnitt 34 in Form eines Gabelkopfes, welcher eine Befestigungsabschnittsaufnahme 36 für einen der Befestigungsabschnitte 20, 22 eines Verbindungselements 18 umfasst. Mittels einer Klemmschraube 38 kann ein in die Befestigungsabschnittsaufnahme 36 eingeführter Befestigungsabschnitt 20, 22 in einer Implantationsstellung klemmend fixiert werden. Durch Festlegen des Befestigungsabschnitts 20, 22 am Halteabschnitt 34 wird vorzugsweise auch eine Relativstellung zwischen dem Knochenverankerungsabschnitt 26 und dem Halteabschnitt 34 dauerhaft festgelegt.

Die Befestigungsabschnitte 20, 22 sind jeweils rundstabförmig ausgebildet und weisen somit einen kreisförmigen Querschnitt auf. Sie sind einstückig mit dem Zwischenabschnitt 24 ausgebildet. Vorzugsweise, wie bei dem in den Figuren dargestellten Ausführungsbeispiel eines Verbindungselements 18, definieren die Befestigungsabschnitte 20 und 22 eine gemeinsame Längsachse 40, die auch eine Längsachse des Zwischenabschnitts 24 definiert.

Der Zwischenabschnitt 24 ist an ein erstes Ende des Befestigungsabschnitts 20 angeformt und bildet im Übergangsbereich 42 zum Befestigungsabschnitt 20 eine im Wesentlichen flache, quaderförmige Endplatte 44 aus. An einer Längsseite derselben schließt sich quer ab von der Längsachse 40 ein gekrümmter Abschnitt 46 an. Dieser erstreckt sich über einen Winkelbereich von etwas mehr als 180°. An den gekrümmten Abschnitt 46 schließt sich ein flacher ebener Abschnitt 48 an, der wiederum in einen gekrümmten Abschnitt 50 übergeht, welcher ebenfalls von der Längsachse 40 weg weisend konvex gekrümmt ist. Die gekrümmten Abschnitte 46 und 50 weisen jedoch in zueinander entgegengesetzte Richtungen. Eine in Richtung auf den zweiten Befestigungsabschnitt 22 hin weisende Endfläche 52 der Endplatte 44 ist etwas gegen den ebenen Abschnitt 48 geneigt. An den gekrümmten Abschnitt 50 schließt sich wiederum ein ebener Abschnitt 54 an, welcher ebenfalls etwas gegen die Endfläche 52 geneigt verläuft. Die schlangenförmige Kontur des Zwischenabschnitts 24 setzt sich fort mit einem weiteren gekrümmten Abschnitt 46, einem weiteren, sich daran anschließenden ebenen Abschnitt 48, einem sich daran anschließenden gekrümmten Abschnitt 50, einem sich daran anschließenden ebenen Abschnitt 54 und einem letzten, sich daran anschließenden gekrümmten Abschnitt 46, welcher in eine zur Endplatte 44 entsprechend geformte Endplatte 56 übergeht, welche eine in Richtung des ersten Befestigungsabschnitts 20 weisende Endfläche 58 aufweist, die ebenfalls etwas geneigt relativ zu den ebenen Abschnitten 54, aber parallel zur Endfläche 52 verläuft.

Eine Steifigkeit des Zwischenabschnitts 24 liegt je nach Wahl des Materials, aus welchem das Verbindungselement 18 hergestellt ist, in einem Bereich von etwa 30 N/mm bis etwa 150 N/mm. Der Zwischenabschnitt 24 ist aus einem insgesamt im Wesentlichen flachen blattfederförmigen Material gebildet beziehungsweise aus einem Vollmaterial durch spanabhebende Bearbeitung, beispielsweise Fräsen, oder Erodieren geformt. Quer ab von der Längsachse 40 weist der Zwischenabschnitt 24 zwei in einer Grundstellung, in welcher auf den Zwischenabschnitt 24 keine äußeren Kräfte einwirken, parallel zueinander und voneinander weg weisende Seitenflächen 60 und 62 auf. Diese verlaufen zudem parallel zu einer die Längsachse 40 enthaltenden Symmetrieebene 64 des Verbindungselements 18, welche eine Spiegelebene 65 definiert.

Durch die schlangenförmige Ausgestaltung des Zwischenabschnitts 24 sind bei dem in den Figuren dargestellten Ausführungsbeispiel insgesamt fünf Ausnehmungen 66, 68 ausgebildet, wobei die drei Ausnehmungen 66 in dieselbe Richtung weisen wie die, in welche die konvex gekrümmten Abschnitte 50 weisend geöffnet sind, die zwei Ausnehmungen 68 jedoch in die entgegengesetzte Richtung, also in die Richtung, in die die konvex gekrümmten Abschnitte 46 weisen. Jede der Ausnehmungen 66, 68 definiert eine Einführöffnung 70 beziehungsweise 72, die jeweils einem gekrümmten Abschnitt 46 beziehungsweise 50 gegenüberliegt und in die jeweils entgegengesetzte Richtung weist. Jede Ausnehmung 66, 68 wird durch zwei in Richtung der jeweiligen Einführöffnung 70, 72 aufeinander zu laufende ebene Abschnitte 48 und 54 begrenzt, so dass sich ein Querschnitt der jeweiligen, in etwa tropfenförmigen Ausnehmung 66, 68 von der Einführöffnung 70 beziehungsweise 72 in Richtung auf die Ausnehmungen 66, 68 weiter begrenzenden gekrümmten Abschnitte 46, 50 hin zunimmt. Damit definiert jede Einführöffnung 70, 72 eine Engstelle 74. Die Ausnehmungen 66, 68 sind somit jeweils in einer Richtung quer zur Längsachse 40 seitlich geöffnet.

Eine Dicke 76 des Zwischenabschnitts 24 im Bereich der gekrümmten Abschnitte 46, 50 ist größer als im Bereich der ebenen Abschnitte 48, 54. Die Dicke 76 beträgt etwa das 1,1fache bis etwa 1,5fache der Dicke 78, vorzugsweise etwa das 1,3fache bis etwa 1,35fache. Bei dem in den Figuren dargestellten Ausführungsbeispiel beträgt die Dicke 76 etwa 0,8 mm, die Dicke 78 etwa 0,6 mm. Die Dicke 76 liegt vorzugsweise in einem Bereich von etwa 0,7 mm bis etwa 0,9 mm, die Dicke 78 in einem Bereich von etwa 0,5 mm bis etwa 0,7 mm. Ein Innenradius der gekrümmten Abschnitte 46, 50 beträgt bei den in den Figuren dargestellten Ausführungsbeispielen etwa 1,6 mm, ein Außenradius etwa 2,2 mm. Beide Radien können abhängig von der Dicke 76 entsprechend abweichen.

Zur Erhöhung der Dauerfestigkeit des Verbindungselements 18 kann dessen äußere Oberfläche 80 durch ein Strahlverfahren mindestens teilweise bearbeitet sein.

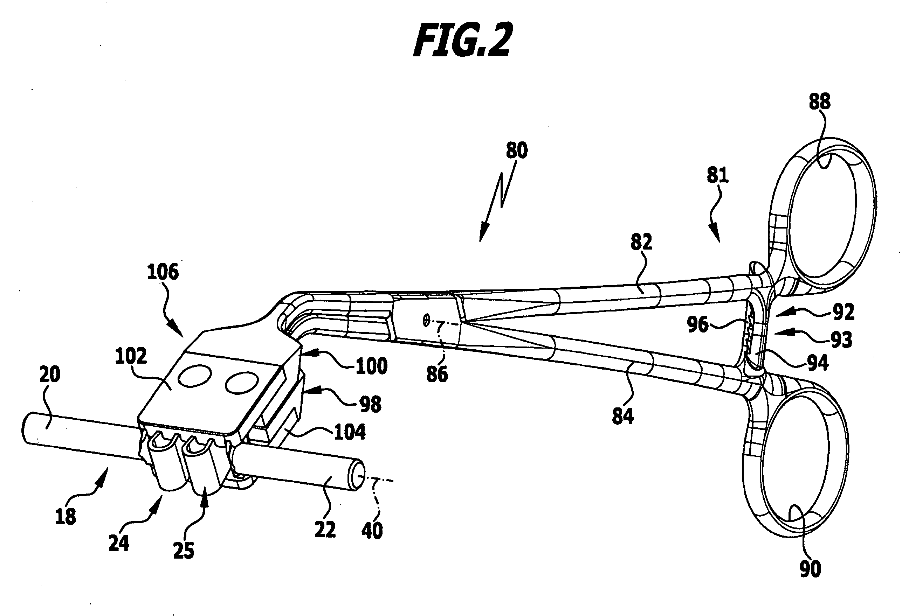

Um das Verbindungselement 18 mit den Befestigungsabschnitten 20 und 22 in definierter Weise in die Befestigungsabschnittaufnahmen 36 einführen und mittels der Klemmschrauben 38 festlegen zu können, dient ein in Figur 2 dargestelltes und insgesamt mit dem Bezugszeichen 80 versehenes chirurgisches Instrument. Es umfasst eine Betätigungseinrichtung 81 mit zwei langgestreckten Branchen 82 und 84, die relativ zueinander um eine Schwenkachse 86 verschwenkbar gelagert sind. Proximale Enden der Branchen 82 und 84 bilden Fingerringe 88 und 90. Etwas distalseitig der Fingerringe 88 und 90 ist eine Sperreinrichtung 92 mit zwei in Eingriff bringbaren Sperrgliedern 94 und 96 schematisch dargestellt, die einerseits an der Branche 82 und andererseits an der Branche 84 angeordnet sind. Die Sperreinrichtung 92, auch als Sperre bezeichnet, verhindert eine Bewegung der Fingerringe 88 und 90 voneinander weg, wenn die Sperrglieder 94 und 96 miteinander in Eingriff stehen. Die Branchen 82 und 84 kreuzen sich im Bereich der Schwenkachse 86. Sie sind weiter distalseitig etwas gekrümmt und gehen beide jeweils in einen plattenförmigen, jeweils einen Rücksprung aufweisenden Haltebackenträger 98 beziehungsweise 100 über.

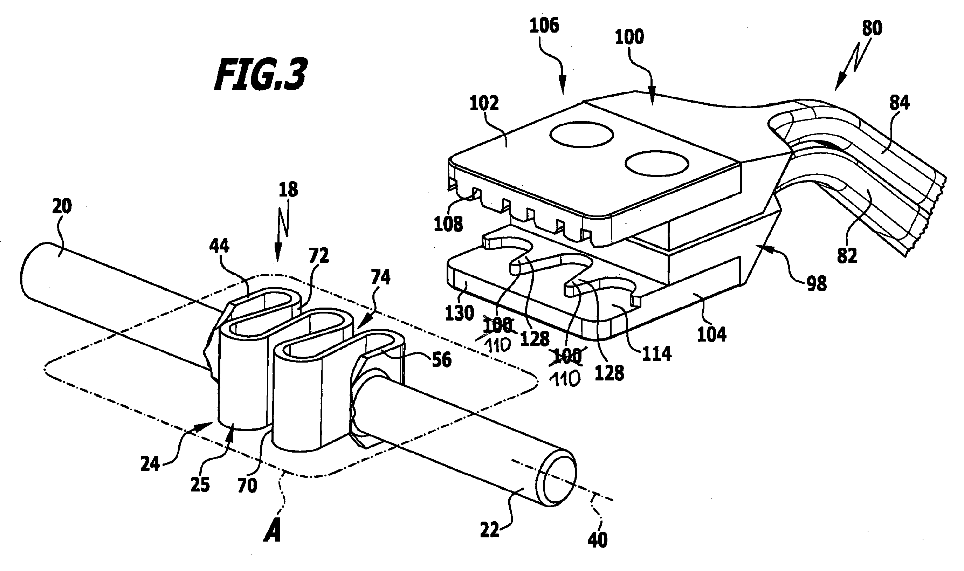

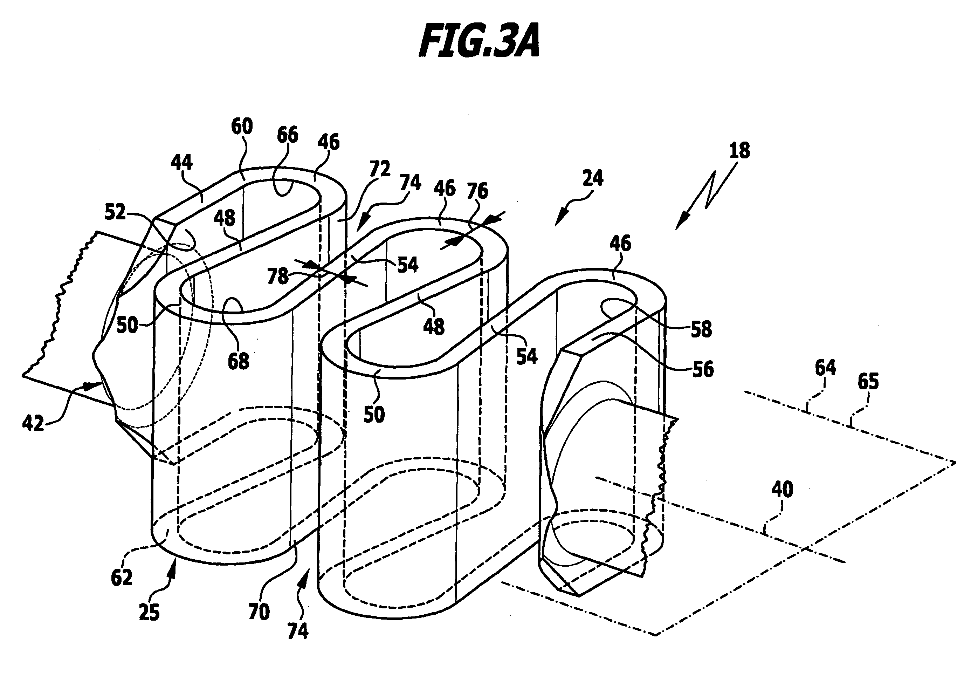

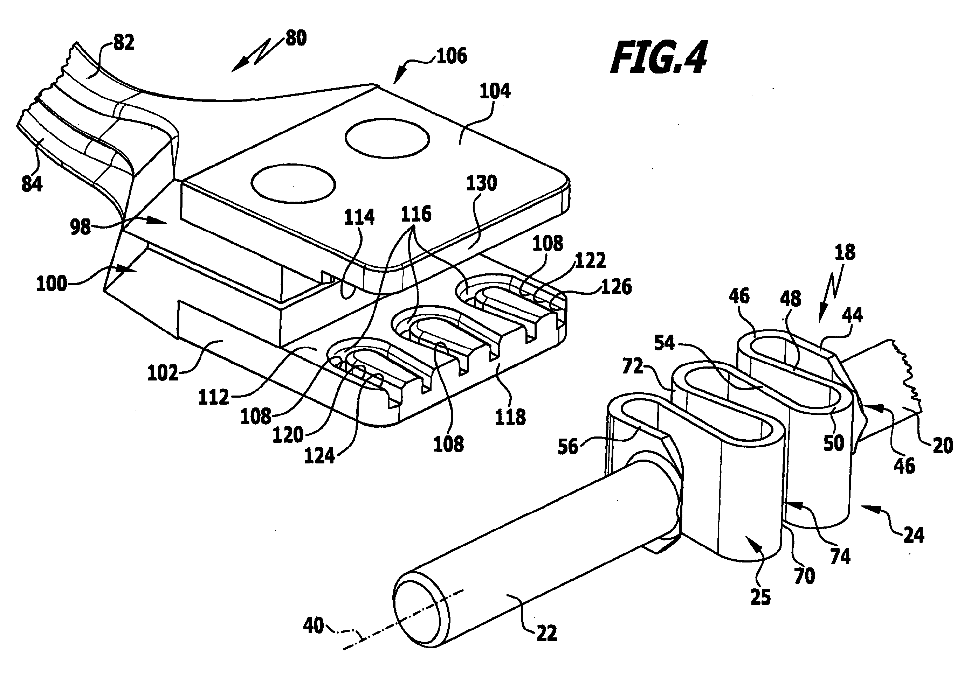

Jeder der beiden Haltebackenträger 98 und 100 trägt einen Haltebacken 102 beziehungsweise 104. Werden die beiden Branchen 82 und 84 gegeneinander geschwenkt, liegen die Haltebackenträger 98 und 100, wie in den Figuren 2 bis 4 schematisch dargestellt, aneinander an. Mit der Sperreinrichtung 92 kann diese Stellung blockiert werden. Sie bildet also eine Verriegelungseinrichtung 93 zum Verhindern einer Bewegung der Haltebacken 102 und 104 voneinander weg aus einer Blockierstellung.

Die Haltebacken 102 und 104 definieren eine chirurgische Vorrichtung 106 zum temporären Versteifen des flexiblen Zwischenabschnitts 24 des Verbindungselements 18. Jeder Haltebacken 102 und 104 umfasst hierfür mehrere Blockierelemente 108 beziehungsweise 110, welche mindestens teilweise formschlüssig mit dem Zwischenabschnitt 24 in Eingriff bringbar sind zum temporären Verhindern einer Verformung des flexiblen Zwischenabschnitts 24. Die Haltebacken 102 und 104 definieren jeweils aufeinander zu weisende ebene Haltefläche 112 und 114.

Der Haltebacken 102 weist drei identische Blockierelemente 108 in Form von am Haltebacken 102 in der Haltefläche 112 vorgesehenen Blockierausnehmungen 116 zum teilweisen Aufnehmen des Blattfederelements 25, und zwar im Bereich der gekrümmten Abschnitte 46, auf. Die Blockierungsausnehmungen 116 sind symmetrisch nebeneinander angeordnet, entsprechend der Form des Blattfederelements 25 ausgebildet und definieren jeweils eine im Wesentlichen U-förmige Nut, die in Richtung auf eine Stirnseite 118 des Haltebackens 102 hin geöffnet ist. Im Wesentlichen aufeinander zu weisende Seitenflächen 120 und 122 der beiden äußeren Blockierausnehmungen 116 verlaufen im Wesentlichen quer zur Längsachse 40 und bilden Axialanschläge 124 und 126, an welchen die Endplatten 44 und 56 anschlagen. Steht das Blattfederelement 25 in Eingriff mit den Blockierausnehmungen 116 des Haltebackens 102, so ist das Blattfederelement 25 im Wesentlichen in Richtung der Längsachse 40, also in axialer Richtung, versteift.

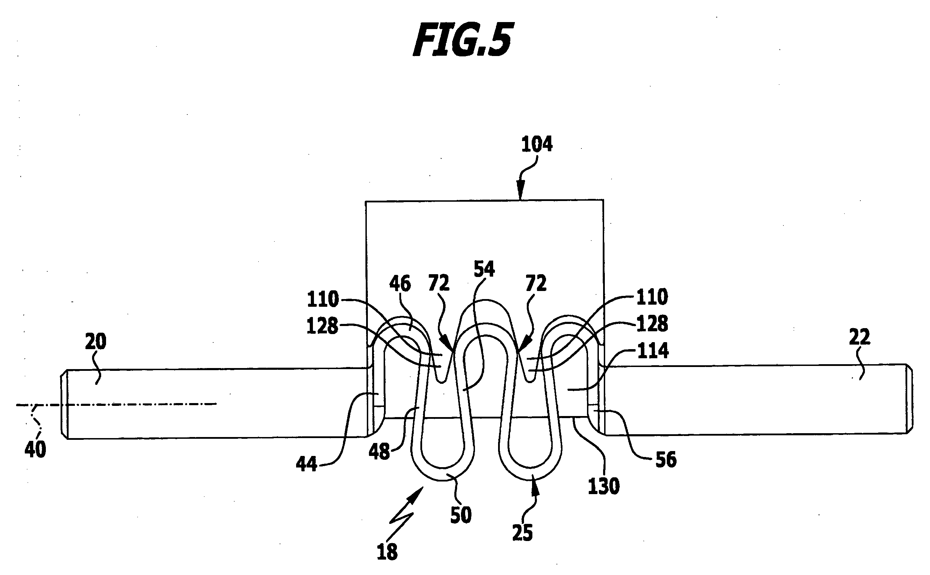

Der Haltebacken 104 weist zwei Blockierelemente 110 in Form zahnartiger Blockiervorsprünge 128 auf. Diese sind auf der Haltefläche 114 von dieser abstehend erhaben ausgebildet und weisen mit ihren Spitzen in distaler Richtung auf eine Stirnkante 130 des Haltebackens 104 hin. Die Blockiervorsprünge 128 sind derart ausgebildet, dass sie, wie in Figur 5 schematisch dargestellt, in die Einführöffnungen 72 zwischen die gekrümmten Abschnitte 46 eingreifen und somit teilweise in die Ausnehmungen 68 hineinragen. Dadurch wird eine Verformung des Blattfederelements 25 zusätzlich blockiert. Die Seitenfläche 60 liegt dabei direkt an der Haltefläche 114 an, die Seitenfläche 62 des Blattfederelements 25 mit einem Teil derselben liegt an in Richtung auf die Haltefläche 114 hin weisenden Nutböden der nutförmigen Blockierausnehmungen 116 an. Das Blattfederelement 25 ist somit zwischen den Haltebacken 102 und 104 gehalten, welche eine Bewegung des Blattfederelements 25 in einer Ebene quer zur Längsachse 40 verhindern. Damit sind alle Freiheitsgrade des Blattfederelements 25 durch die Vorrichtung 106 blockiert. Das Verbindungselement 18 ist somit temporär versteift und das Blattfederelements 25 an einer Verformung gehindert.

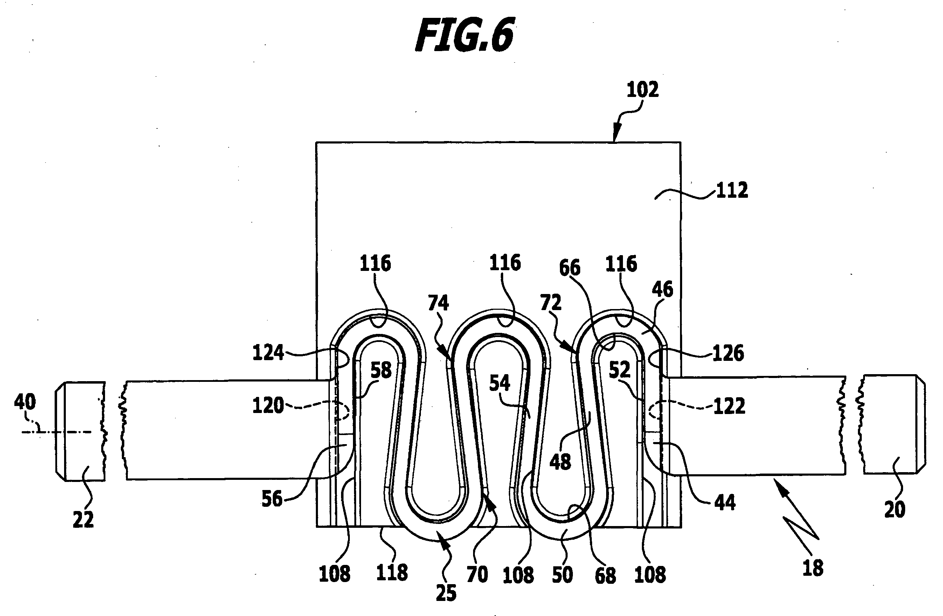

Ein Operateur kann mit dem Instrument 80 das Verbindungselement 18 in der beschriebenen und in den Figuren 2 bis 6 dargestellten Weise ergreifen oder fassen, das Blattfederelement 25 temporär versteifen und es mittels des Instruments 80 in die Befestigungsabschnittaufnahmen 36 der Knochenbefestigungseinrichtungen 12 und 14 einsetzen. Das Verbindungselement 18 kann insbesondere so lange mit dem Instrument 80 gehalten werden, bis die Befestigungsabschnitte 20 und 22 mit den Klemmschrauben 38 an den Knochenbefestigungseinrichtungen 12 und 14 in gewünschter Weise festgelegt sind. Sobald dies der Fall ist, kann der Operateur die gegebenenfalls aktivierte Sperreinrichtung 92 wieder lösen und die beiden Haltebacken 102 und 104 voneinander weg verschwenken, um das Blattfederelement 25 wieder freizugeben. Durch die schwenkbare Lagerung der Branchen 82 und 84 um die Schwenkachse 86 sind auch die Haltebacken 102 und 104 relativ zueinander in einer Bewegungsrichtung bewegbar, bei dem in den Figuren 2 bis 5 dargestellten Ausführungsbeispiel sind sie gegeneinander verschwenkbar.

Die Sperreinrichtung 92 bildet auf diese Weise eine Verriegelungseinrichtung 93 zum Verhindern einer Bewegung der Haltebacken 102 und 104 in einer Bewegungsrichtung voneinander weg, wenn die Haltebacken 102 und 104 eine Blockierstellung einnehmen, wie schematisch in den Figuren 2 bis 4 dargestellt.

Die Haltebacken 102 und 104 können optional die Vorrichtung 106 auch ohne die Betätigungseinrichtung 81 ausbilden. In diesem Fall werden die Haltebacken 102 und 104 entweder direkt am Zwischenabschnitt 24 befestigt oder aneinander, wenn sie mit dem Blattfederelement 25 in Eingriff stehen, beispielsweise mit einem nicht dargestellten Faden oder einer Klammer.

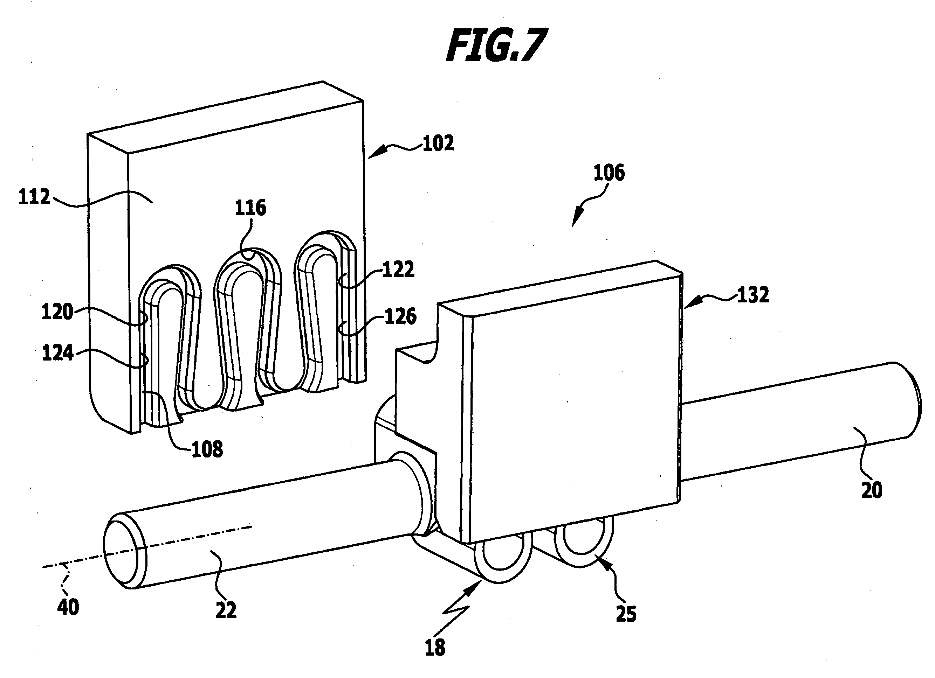

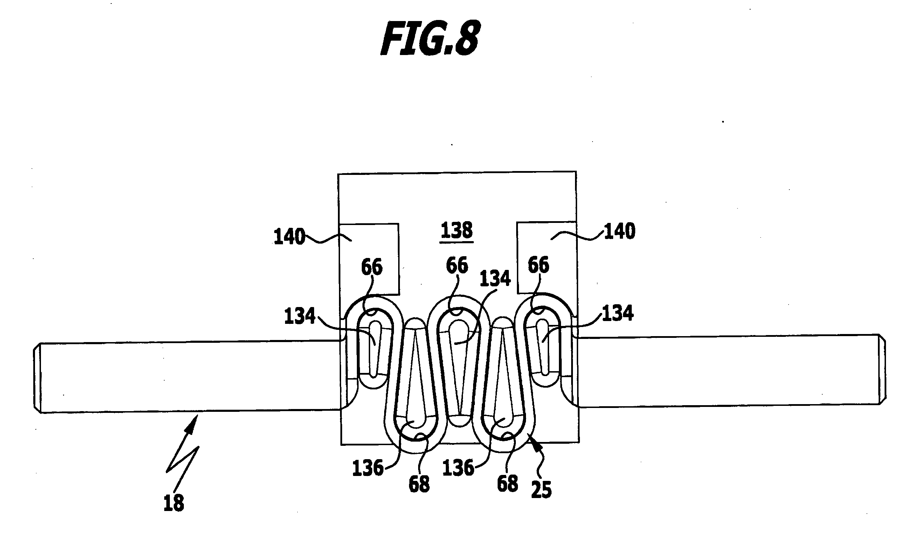

In den Figuren 7 und 8 ist ein weiteres Ausführungsbeispiel einer chirurgischen Vorrichtung 106 dargestellt. Diese umfasst einen Haltebacken 102 sowie einen Haltebacken 132, welcher ähnlich dem Haltebacken 104 ausgebildet ist. Der Haltebacken 132 weist insgesamt fünf Blockiervorsprünge 134, 136 auf, wobei die drei Blockiervorsprünge 134 korrespondierend zu den Ausnehmungen 66 ausgebildet sind, die beiden Blockiervorsprünge 136 korrespondierend zu den Ausnehmungen 68. Sie stehen von einer Haltefläche 138 ab, welche in Richtung auf die Haltefläche 112 des Haltebackens 102 hin weist. Ferner stehen vom Haltebacken 132, das heißt von dessen Haltefläche 138, zwei im Wesentlichen aufeinander zu weisende, Axialanschläge 140 definierende Vorsprünge ab, an denen jeweils ein gekrümmter Abschnitt 46 teilweise anliegt, und zwar im Bereich des Übergangs zu den beiden Endplatten 44 und 56. Der Haltebacken 132 in Verbindung mit dem Haltebacken 102 ermöglicht so eine vollständige Versteifung des Zwischenabschnitts 24, wenn sie mit dem Blattfederelement 25 in Eingriff stehen.

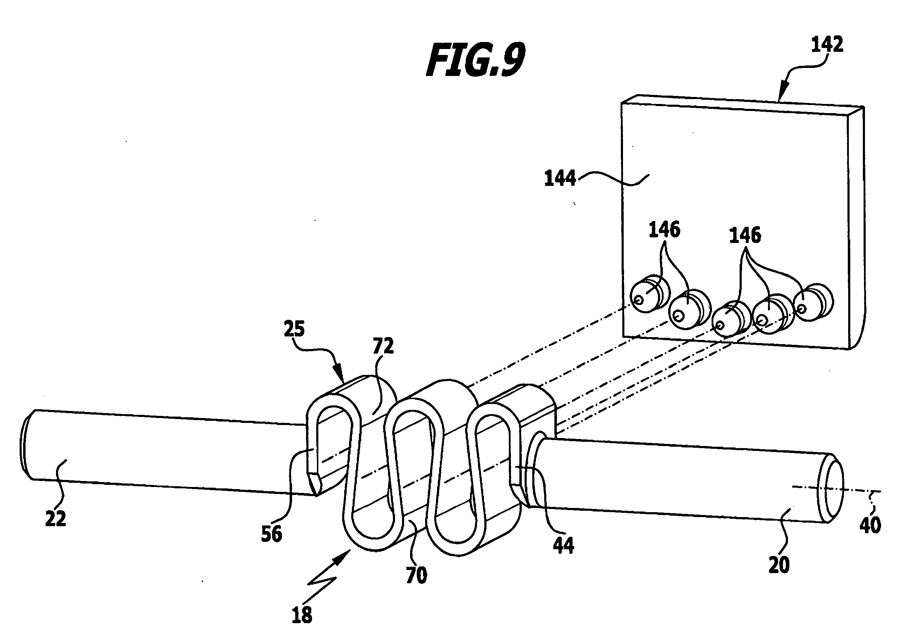

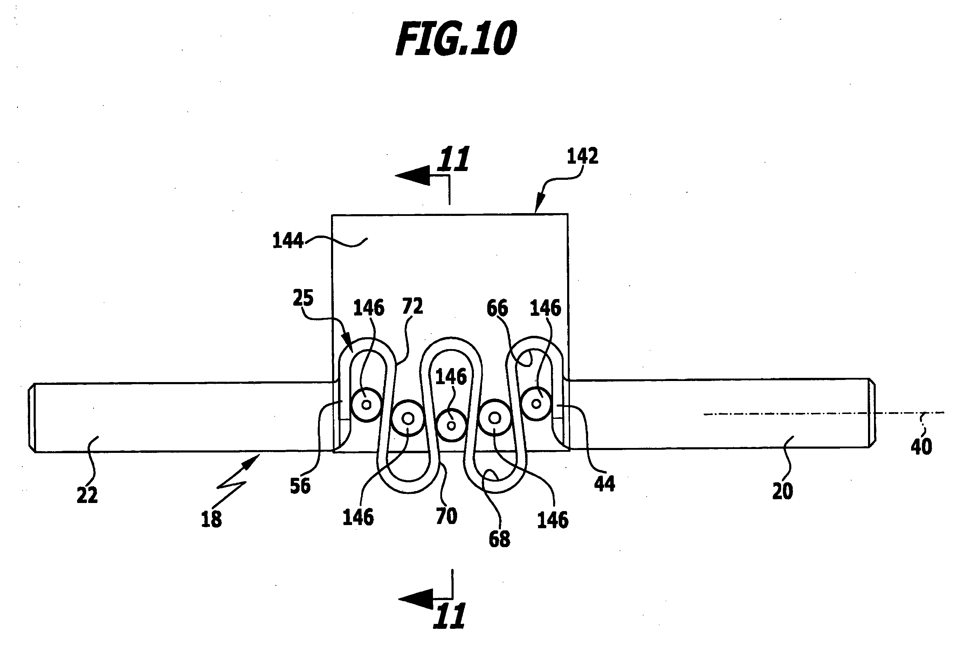

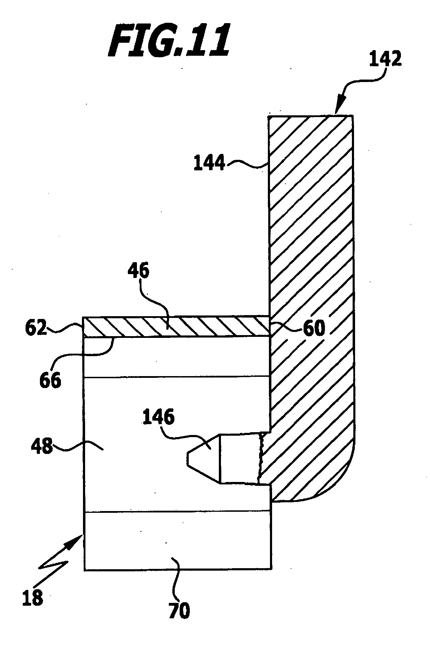

Statt des Haltebackens 104 kann beispielsweise ein in den Figuren 9 bis 11 dargestellter Haltebacken 142 verwendet werden. Dieser umfasst eine quaderförmige Platte, welche eine Haltfläche 144 definiert und von der insgesamt fünf Blockierelemente 146 in Form von im Wesentlichen zylindrischen Blockiervorsprüngen abstehen, die derart angeordnet sind, dass sie in die Ausnehmungen 66 und 68 eingreifen, und zwar jeweils zwischen zwei geradlinige Abschnitte 48 des Blattfederelements 25 beziehungsweise zwischen einen geradlinigen Abschnitt 48 und eine der beiden Endplatten 44 und 56. In Verbindung mit einem weiteren Haltebacken, welcher in Form eines der Haltebacken 102, 104 oder 132 ausgebildet sein kann, lässt sich so ebenfalls eine chirurgische Vorrichtung 106 zum temporären Versteifen des Zwischenabschnitts 24 ausbilden. Selbstverständlich kann der Haltebacken 142 wie alle oben beschriebenen Haltebacken auch, einen Teil des Instruments 80 bilden.

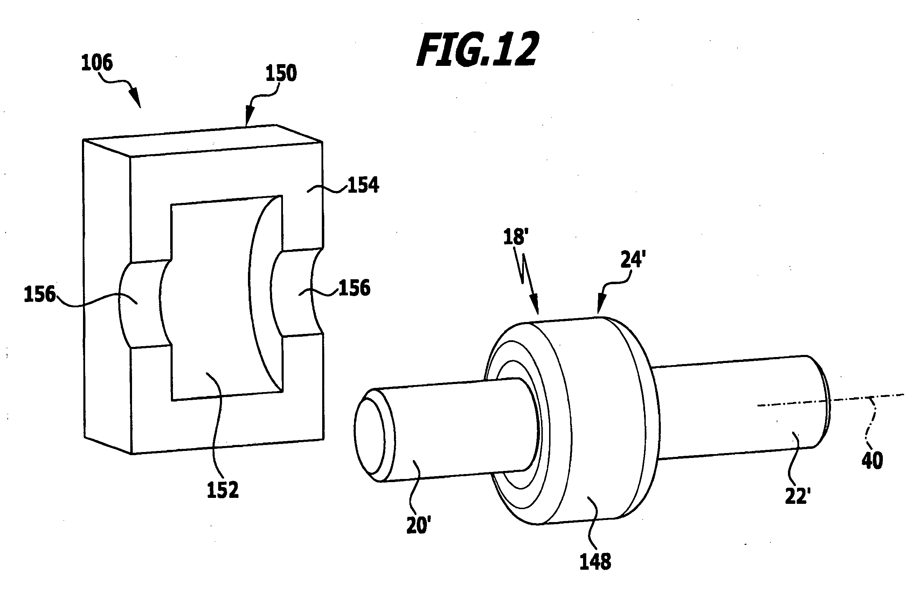

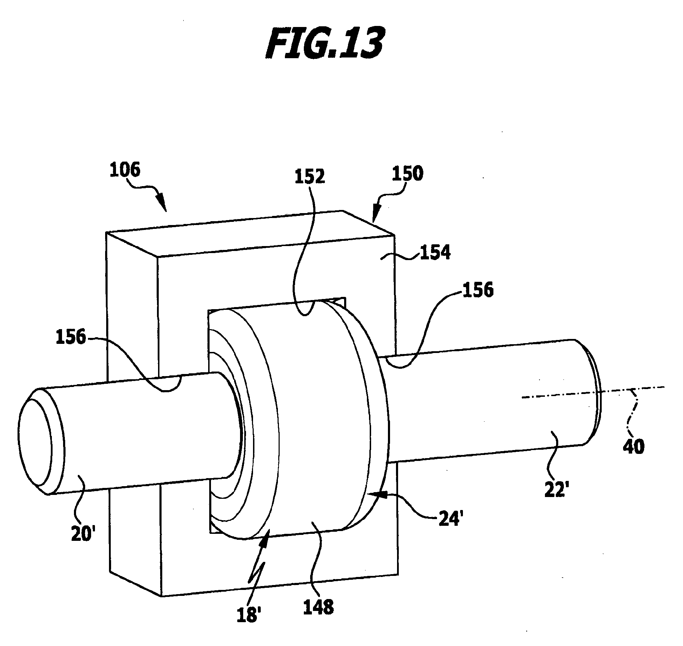

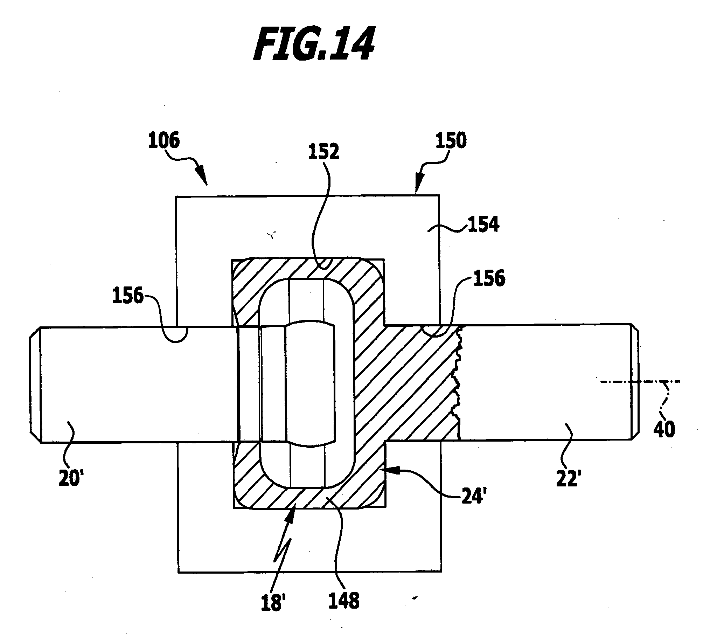

In den Figuren 12 bis 14 ist ein insgesamt mit dem Bezugszeichen 18' versehenes, nicht erfindungsgemäßes Verbindungselement dargestellt, welches zwei stabförmige Befestigungsabschnitte 20' und 22' umfasst, welche über ein hohlzylindrisches Kupplungselement miteinander gekuppelt sind. Das Kupplungselement 148 ist teilweise flexibel beziehungsweise verformbar ausgebildet und ermöglicht eine gedämpfte Bewegung der Befestigungsabschnitte 20' und 22' relativ zueinander sowohl in Richtung einer Längsachse 40 des Verbindungselements 18' als auch quer zu dieser. Zum Verhindern einer Bewegung der Befestigungsabschnitte 20' und 22' relativ zueinander kann das einen Zwischenabschnitt 24' definierende Kupplungselement 148 mittels einer weiteren Variante einer chirurgischen Vorrichtung 106 temporär versteift werden. Hierzu dienen vorzugsweise zwei identisch ausgebildete Haltebacken 150, welche jeweils eine Blockierausnehmung 152 aufweisen, die die Form eines halben Zylinders aufweist und somit das Kupplungselement 148 zur Hälfte formschlüssig aufnehmen kann. Die Blockierausnehmung 152 ist in einer Haltefläche 154 des Haltebackens 150 ausgebildet. Zur Aufnahme der Befestigungsabschnitte 20' und 22' schließen sich an die Biockierausnehmung 152 halbschalige Stabaufnahmen 156 an.

Mit den beiden identischen Haltebacken 150 kann somit das Kupplungselement 148 im Wesentlichen vollständig umschlossen werden, so dass der Zwischenabschnitt 24' an einer Verformung in allen Raumrichtungen gehindert ist.

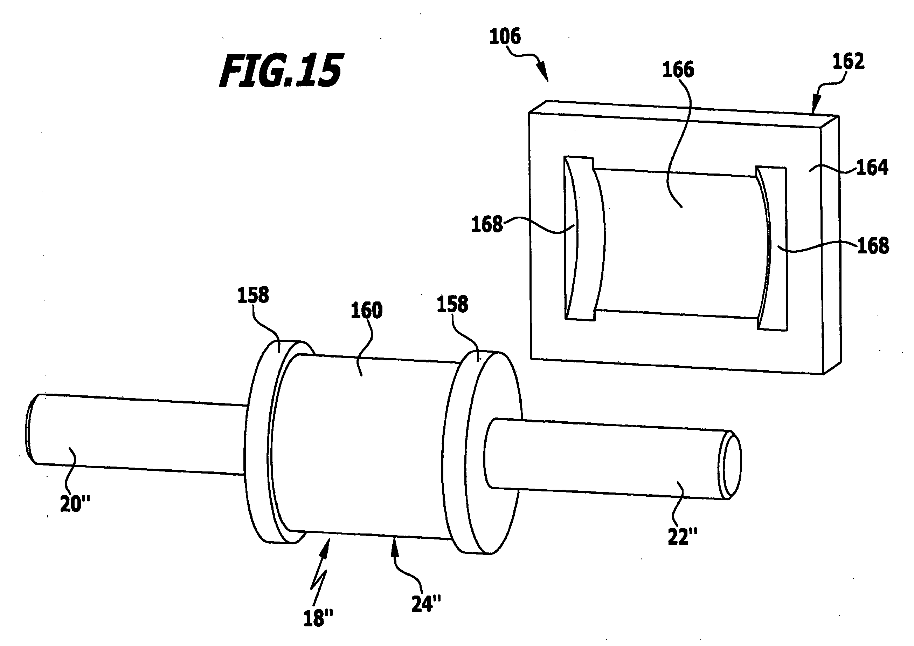

In Figur 15 ist schematisch ein weiteres, nicht erfindungsgemäßes Verbindungselements 18" dargestellt, welches zwei stabförmige Befestigungsabschnitte 20" und 22" umfasst, welche jeweils an einem Ende mit einer scheibenförmigen Trägerplatte 158 ausgestattet sind, die aufeinander zu weisen. Zwischen den Trägerplatten 158 ist ein elastisches Element 160 angeordnet, welches eine zylindrische Form ausweist. Der Zwischenabschnitt 24" wird somit gebildet durch die beiden Trägerplatten 158 und das zwischen diesen gehaltene elastische Element 160. Ein Außendurchmesser der Trägerplatten 158 ist etwas größer als der des elastischen Elements 160.

Zur Ausbildung einer weiteren chirurgischen Vorrichtung 106 sind vorzugsweise zwei identische Haltebacken 162 vorgesehen, von denen einer in Figur 15 schematisch dargestellt ist. Er ist in Form einer quaderförmigen Platte ausgebildet und definiert eine ebene Haltefläche 164. Von der Haltefläche 164 ausgehend in den Haltebacken 162 hinein ist eine Blockierausnehmung 166 ausgebildet, welche den Zwischenabschnitt 24" teilweise formschlüssig aufnehmen kann. Die Blockierausnehmung 166 ist somit im Wesentlichen korrespondierend zu einem Teil, beispielsweise einer Hälfte, des Zwischenabschnitts 24" ausgebildet. Mit zwei Haltebacken 162 lässt sich der flexible Zwischenabschnitt 24" temporär versteifen. Aufeinander zu weisende Seitenflächen 168 der Blockierausnehmung 166 bilden Axialanschläge für den Zwischenabschnitt 24". Ist der Zwischenabschnitt 24" in der Blockierausnehmung 166 gehalten, liegen die Trägerplatten 158 jeweils an den Seitenflächen 168 der beiden Haltebacken 162 an. Durch die beiden Haltebacken 162 gemeinsam können alle Bewegungsfreiheitsgrade des Verbindungselements 18" blockiert werden.

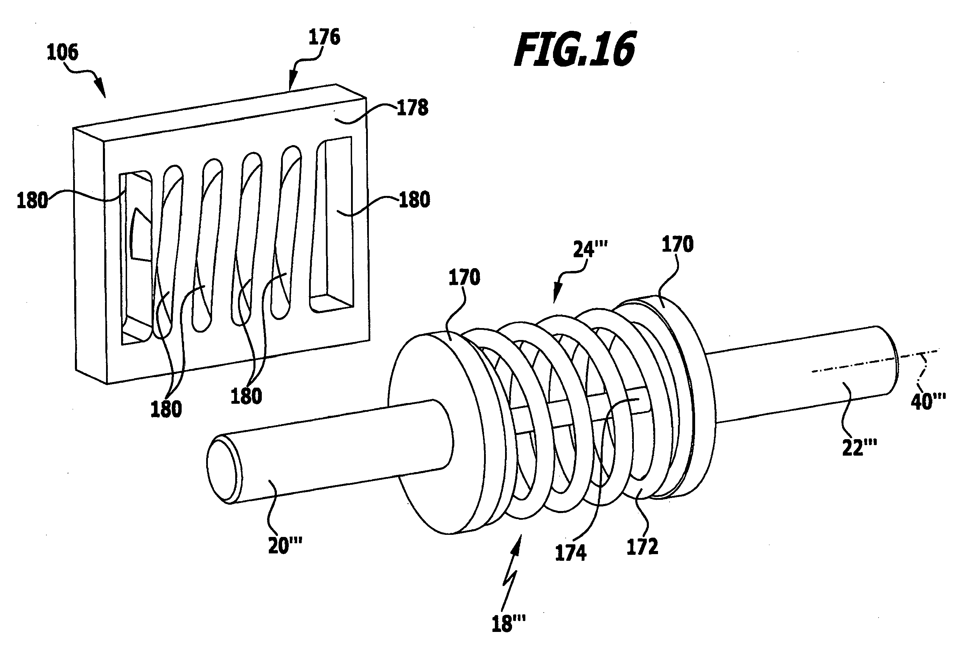

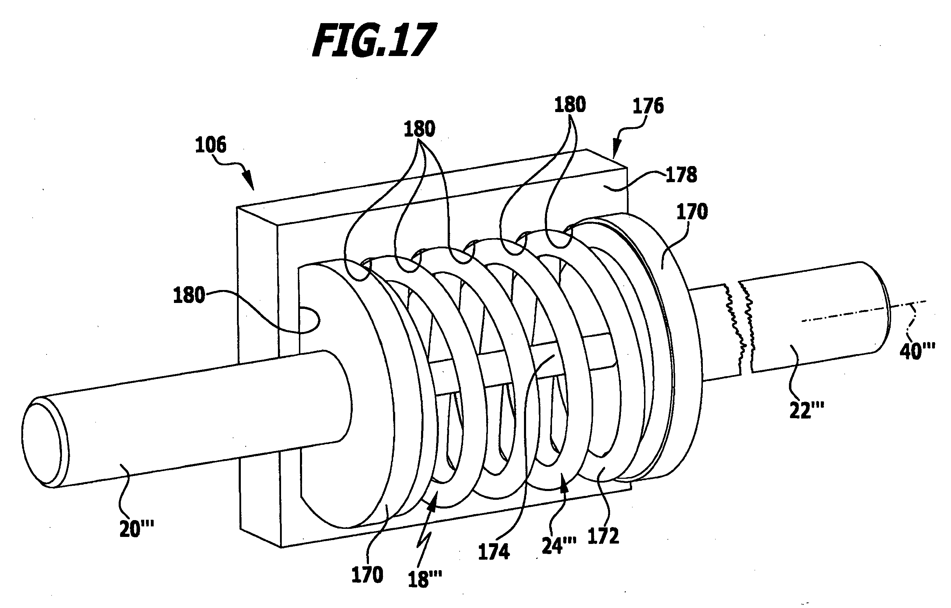

In den Figuren 16 und 17 ist schematisch ein weiteres Ausführungsbeispiel eines insgesamt mit dem Bezugszeichen 18"' bezeichneten Verbindungselements dargestellt. Es umfasst zwei stabförmige Befestigungsabschnitte 20'" und 22"', die jeweils an einem freien Ende eine scheibenförmige Trägerplatte 170 aufweisen, die aufeinander zu weisend angeordnet sind. Zwischen den Trägerplatten 170 ist eine im Außendurchmesser an den Außendurchmesser der Trägerplatten 170 angepasste Schraubenfeder 172 angeordnet. Des Weiteren ist von einer Trägerplatte 170 senkrecht abstehend ein Führungsstab 174 vorgesehen, welcher in eine Bohrung der gegenüberliegenden Trägerplatte 170 eingreift, die sich im zugehörigen Befestigungsabschnitt 20'" oder 22"' entsprechend fortsetzt. Durch diese besondere Ausgestaltung ist es möglich, die Trägerplatten 170 entgegen der Wirkung der Schraubenfeder 172 aufeinander zu zu bewegen. Eine Rotation der Trägerplatten 170 um die Längsachse ist zwar unter Umständen möglich, aufgrund des vorgesehenen Führungsstabs 174 ist jedoch keine Bewegung in einer Richtung quer zur Längsachse möglich.

In den Figuren 16 und 17 ist schematisch ein weiterer Haltebacken 176 dargestellt, welcher zusammen mit einem identisch ausgebildeten weiteren Haltebacken 176 eine weitere chirurgische Vorrichtung 106 ausbildet zum temporären Versteifen des Zwischenabschnitts 24"', welcher gebildet wird durch die Trägerplatten 170 in Verbindung mit der Schraubenfeder 172. Der quaderförmige Haltebacken 176 weist eine ebene Haltefläche 178 auf, in der mehrere Blockierausnehmungen 180 ausgebildet sind zum teilweisen Aufnehmen der Trägerplatten 170 sowie der Schraubenfeder 172. Wird in analoger Weise wie in Figur 17 dargestellt ein weiterer Haltebacken 176 mit dem Zwischenabschnitt 24"' von der Seite, also quer zur Längsachse 40"', in Eingriff gebracht, kann der Zwischenabschnitt 24'" temporär versteift werden.

Die oben beschriebenen Haltebacken 150, 162 und 176 können auch in der oben beschriebenen Weise einen Teil eines chirurgischen Instruments 80 bilden, um die jeweiligen Verbindungselemente temporär zu versteifen. Sie können jedoch auch direkt miteinander gekuppelt oder anderweitig verbunden werden, beispielsweise mit einem Faden oder einer Kopplungseinrichtung, um die Verbindungselemente beim Implantieren an einer Verformung zu hindern.

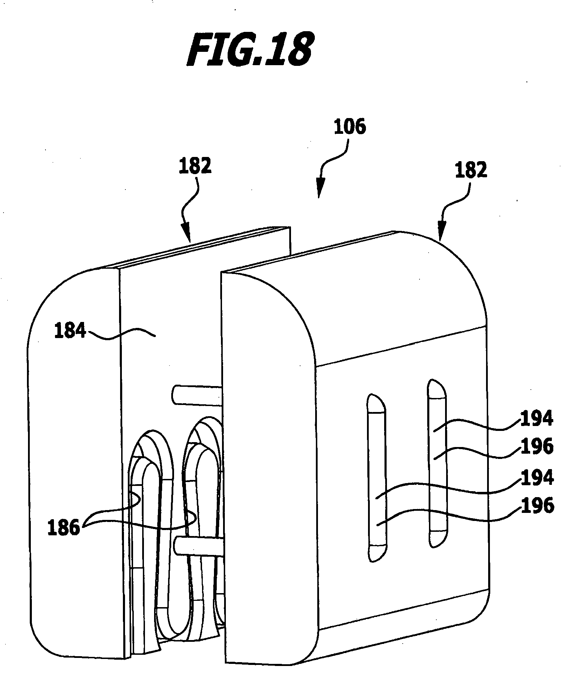

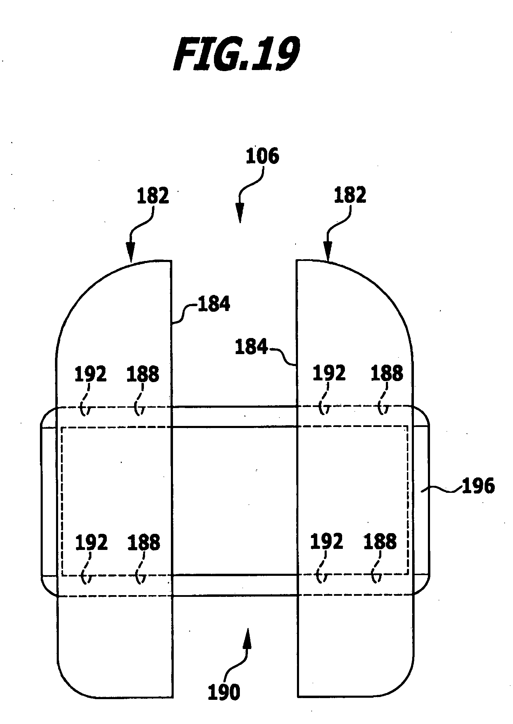

In den Figuren 18 und 19 ist eine weitere Variante einer chirurgischen Vorrichtung 106 dargestellt, die zwei identische Haltebacken 182 umfasst. In aufeinander zu weisenden Halteflächen 184 der Haltebacken 182 sind mehrere Blockierausnehmungen 186 ausgebildet, die den Blockierausnehmungen 116 entsprechen. Dies ermöglicht es, zwischen den Haltebacken 182 beispielsweise das Blattfederelement 25 temporär zu fixieren.

Die beiden Haltebacken 182 sind ferner jeweils mit zwei Bohrungen 188 versehen, die senkrecht zu den Halteflächen 184 orientiert sind. Jeder Bohrung 188 eines Haltebackens 182 liegt eine weitere Bohrung 188 des anderen Haltebackens 182 gegenüber, wobei diese koaxial zueinander ausgerichtet sind, wenn die Blockierausnehmungen 186 mit dem Blattfederelement 25 des nicht dargestellten Verbindungselements 18 in Eingriff stehen. Um die Haltebacken 182 temporär am Zwischenabschnitt 24 zu fixieren, dient eine Kupplungseinrichtung 190 mit zusammenwirkenden ersten Kupplungselementen 192 in Form der Bohrungen 188 und zweiten Kupplungselementen 194 in Form von Fäden oder Drähten, welche ringförmig geschlossen jeweils zwei Bohrungen beider Haltebacken 182 durchsetzt. Durch entsprechendes Anpassen einer Länge der Fäden 196 können die Halteflächen 184 gegeneinander gezogen werden in eine Blockierstellung, in welcher die Seitenflächen 60 und 62 an den aufeinander zu weisenden Nutböden der Blockierausnehmungen 186 anliegen. In dieser Blockierstellung ist dann der Zwischenabschnitt 24 temporär versteift. Zum Lösen der chirurgischen Vorrichtung 106 kann nach endgültiger Positionierung des Verbindungselements 18 an den Knochenschrauben 16 die Kupplungseinrichtung 190 gelöst werden, beispielsweise durch Durchtrennen der zweiten Kupplungselemente 194.

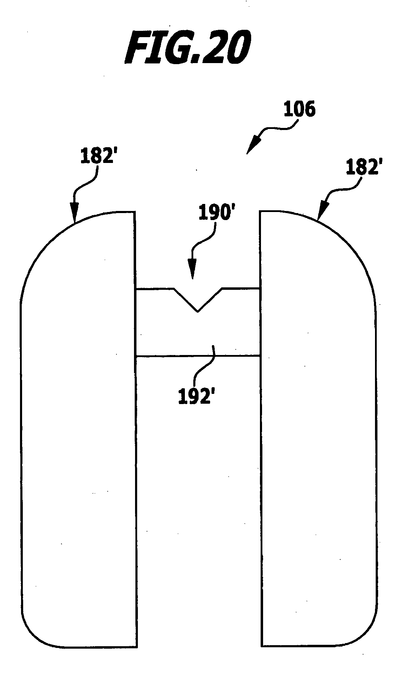

In Figur 20 ist eine chirurgische Vorrichtung 106 schematisch dargestellt, die im Wesentlichen der in den Figuren 18 und 19 dargestellten Vorrichtung 106 entspricht. Sie unterscheidet sich jedoch in der Ausgestaltung der Kupplungseinrichtung 190', welche ein erstes Kupplungselement 192' in Form eines Kupplungsstegs umfasst, welcher in nicht dargestellte Ausnehmungen der Haltebacken 182' eingreift und in einer Kupplungsstellung mit diesen rastend in Eingriff stehen kann.

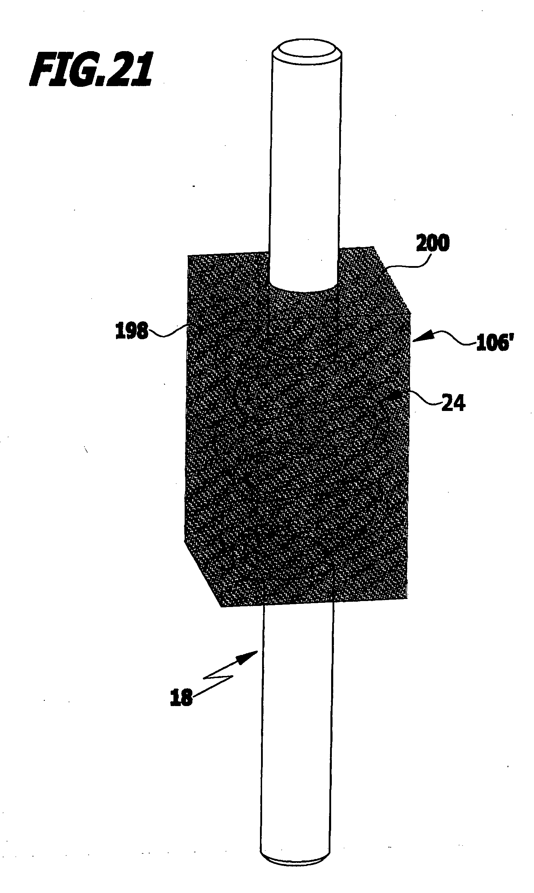

In Figur 21 ist ein weiteres Ausführungsbeispiel einer chirurgischen Vorrichtung 106' schematisch dargestellt, welche zum temporären Versteifen des Verbindungselements 18 oder eines der weiteren, oben beschriebenen Verbindungselemente genutzt werden kann. Die chirurgische Vorrichtung 106' umfasst einen Formkörper 198, welcher eine quaderförmige Außenkontur aufweist und den Zwischenabschnitt 24' vollständig formschlüssig umgibt. Er bildet somit ein Blockierelement 200.

Zur Ausbildung des Formkörpers 198 wird der Zwischenabschnitt 24' mit einem flüssigkeitslöslichen Material eingehüllt oder umhüllt, beispielsweise kann dies ein Formkörper 198 aus Zucker oder Salz sein. Der Formkörper 198 kann auch ein Gemisch aus Zucker und Salz enthalten. Denkbar sind auch andere Materialien, die sich durch eine Flüssigkeit, beispielsweise durch Wasser, auslösen lassen. Das Verbindungselement 18 kann dann zum Beispiel nach seiner Herstellung mit dem Formkörper 198 ausgeliefert werden. Nach der Implantation des Verbindungselements 18 lässt sich zum Entfernen des Formkörpers 198 das Verbindungselement 18 mit einer das Material, aus dem der Formkörper 198 hergestellt ist, lösenden Flüssigkeit umspülen. Nach mindestens teilweiser, vorzugsweise vollständiger Auflösung des den Formkörper 198 ausbildenden Materials ist dann das Verbindungselement 18 in gewünschter Weise beweglich.

Des Weiteren ist es denkbar, den Formkörper 198 aus einem Material herzustellen, dessen Aggregatzustand zum Entfernen der Vorrichtung 106 änderbar ist. Beispielsweise kann der Formkörper 198 aus Eis gebildet werden, das in Folge einer Temperaturänderung schmilzt, also flüssig wird, und somit das Blattfederelement 25 freigibt. Denkbar sind statt Eis auch biokompatible Kunststoffe, die in Folge einer Erwärmung über ihre Fließtemperatur das Blattfederelement 25 ebenfalls zumindest soweit freigeben, dass sich dieses in gewünschter Weise verformen lässt.

Eine weitere Variante zur Ausbildung des Formkörpers 198 besteht darin, diesen aus einem resorbierbaren Kunststoff auszubilden, der beispielsweise nach Herstellung des Verbindungselements 18 an den Zwischenabschnitt 24 angespritzt wird. Nach der Implantation des Verbindungselements 18 kann dann der Kunststoff vom Körper des Patienten resorbiert werden, wobei nach vollständiger Resorption des Formkörpers 198 das Blattfederelement 25 vollständig freiliegt und unter Belastung in gewünschter Weise verformbar ist.

Wird der Formkörper 198 aus einem biokompatiblen Kunststoff hergestellt, lässt sich dieser beispielsweise auch durch Ultraschall vom Blattfederelement 25 lösen.

Insbesondere die Vorrichtung 106', aber auch die anderen, oben beschriebenen chirurgischen Vorrichtungen 106 ermöglichen eine temporäre Einschränkung der Bewegung des Zwischenabschnitts 24. Während der Implantation des jeweiligen Verbindungselements schützen sie den Zwischenabschnitt vor etwaigen Beschädigungen. Durch die beschriebenen chirurgischen Vorrichtungen ist es möglich, die beschriebenen Verbindungselemente in neutraler, das heißt unbelasteter Stellung zu implantieren.

Die im Zusammenhang mit den Figuren beschriebenen Verbindungselemente können aus einem metallischen Material oder einem Kunststoff hergestellt sein. Beispiele für geeignete Materialien sind bereits weiter oben angegeben.

## Patentansprüche

1. Chirurgische Vorrichtung (106) zum temporären Versteifen eines mindestens teilweise flexiblen Zwischenabschnitts (24; 24"') eines Verbindungselements (18; 18"') für ein Wirbelsäulenstabilisierungssystem (10), welches Verbindungselement (18; 18"') einen ersten Befestigungsabschnitt (20) zum Festlegen an einer ersten Knochenbefestigungseinrichtung (12), einen zweiten Befestigungsabschnitt (22) zum Festlegen an einer zweiten Knochenbefestigungseinrichtung (14) und den zwischen dem ersten und dem zweiten Befestigungsabschnitt (20, 22) angeordneten oder ausgebildeten, mindestens teilweise flexiblen Zwischenabschnitt (24; 24"') umfasst, welcher Zwischenabschnitt (24; 24"') mindestens eine Ausnehmung (66, 68) definiert, die derart ausgebildet ist, dass sie eine Verformung des Zwischenabschnitts (24; 24"') ermöglicht, wobei die chirurgische Vorrichtung (106) mindestens ein Blockierelement (108, 110) umfasst, welches mindestens teilweise formschlüssig mit dem Zwischenabschnitt (24; 24"') in Eingriff bringbar ist zum temporären Verhindern einer Verformung des flexiblen Zwischenabschnitts (24; 24"'), **gekennzeichnet durch** mindestens zwei in einer Bewegungsrichtung aufeinander zu und voneinander weg bewegbare Haltebacken (102, 104; 132; 142; 150; 162, 176; 182) mit aufeinander zu weisenden Halteflächen (112, 114; 138; 144; 154; 164; 178; 184), wobei mindestens einer der Haltebacken (102, 104) das mindestens eine Blockierelement (108, 110) trägt oder umfasst.

2. Chirurgische Vorrichtung nach Anspruch 1, **dadurch gekennzeichnet, dass** die mindestens zwei Haltebacken (102, 104) insgesamt mindestens zwei aufeinander zu weisende Axialanschläge (124, 126) umfassen, welche in einer Richtung quer zur Bewegungsrichtung der Haltebacken (102, 104) relativ zueinander wirkend ausgebildet sind zum Verhindern einer Bewegung des Zwischenabschnitts (24) in axialer Richtung (40).

3. Chirurgische Vorrichtung nach Anspruch 1 oder 2, **gekennzeichnet durch** eine Verriegelungseinrichtung (93) zum Verhindern einer Bewegung der Haltebacken (102, 104; 132; 142; 150; 162, 176; 182) in der Bewegungsrichtung voneinander weg in einer Blockierstellung.

4. Chirurgische Vorrichtung nach einem der voranstehenden Ansprüche, **dadurch gekennzeichnet, dass** das mindestens eine Blockierelement (108) in Form eines von einer Haltefläche (112) eines der Haltebacken (102) abstehenden Blockiervorsprungs (128) ausgebildet ist oder dass das mindestens eine Blockierelement (108) in Form einer an einem der Haltebacken (102) in der Haltefläche (112) vorgesehenen Blockierausnehmung (116) zum mindestens teilweisen Aufnehmen des temporär zu versteifenden Zwischenabschnitts (24) ausgebildet ist.

5. Chirurgische Vorrichtung nach Anspruch 4, **dadurch gekennzeichnet, dass** der Blockiervorsprung (128; 134) keilförmig oder im Wesentlichen keilförmig ausgebildet ist.

6. Chirurgische Vorrichtung nach Anspruch 4, **dadurch gekennzeichnet, dass** mindestens einer der Axialanschläge (124, 126) an einer Blockierausnehmung (116) ausgebildet ist.

7. Chirurgische Vorrichtung nach einem der voranstehenden Ansprüche, **dadurch gekennzeichnet, dass** die mindestens zwei Haltebacken (102, 104; 132; 142; 150; 162, 176; 182) unterschiedlich ausgebildet sind.

8. Chirurgische Vorrichtung nach Anspruch 7, **dadurch gekennzeichnet, dass** die Haltebacken (102, 104; 132; 142; 150; 162, 176; 182) unterschiedlich geformte Blockierelemente (108, 110) umfassen.

9. Chirurgische Vorrichtung nach einem der voranstehenden Ansprüche, **gekennzeichnet durch** eine Kupplungseinrichtung (190; 190') zum lösbaren Verbinden der zwei Haltebacken (102, 104; 132; 142; 150; 162, 176; 182) miteinander in einer Kupplungsstellung.

10. Chirurgische Vorrichtung nach Anspruch 9, **dadurch gekennzeichnet, dass** die Kupplungseinrichtung (190; 190') erste und zweite Kupplungs-elemente (192, 194; 192') umfasst, welche einerseits am einen Haltebacken (102, 104; 132; 142; 150; 162, 176; 182) und andererseits am anderen Haltebacken (102, 104; 132; 142; 150; 162, 176; 182) angeordnet oder ausgebildet sind und in der Kupplungsstellung in Eingriff stehen.

11. Chirurgische Vorrichtung nach Anspruch 9 oder 10, **dadurch gekennzeichnet, dass** die Kupplungseinrichtung (190') einen Kupplungssteg (192') oder einen Faden umfasst.

12. Chirurgische Vorrichtung nach einem der voranstehenden Ansprüche, **gekennzeichnet durch** eine Betätigungseinrichtung (81) zum Überführen der Vorrichtung (106) von einer Anlegestellung, in welcher die Vorrichtung (106) und der Zwischenabschnitt (24; 24"') außer Eingriff stehen, in eine Blockierstellung, in welcher mit der Vorrichtung (106) der Zwischenabschnitt (24; 24"') unverformbar oder im Wesentlichen unverformbar an der Vorrichtung (106) gehalten ist.

13. Chirurgische Vorrichtung nach Anspruch 12, **dadurch gekennzeichnet, dass** die Betätigungseinrichtung (81) zwei relativ zueinander bewegbare Betätigungsglieder (82, 84) umfasst, welche direkt oder indirekt mit den mindestens zwei Haltebacken (102, 104; 132; 142; 150; 162, 176; 182) gekoppelt sind zum Bewegen derselben.

14. Wirbelsäulenstabilisierungssystem (10) umfassend mindestens eine erste Knochenbefestigungseinrichtung (12), mindestens eine zweite Knochenbefestigungseinrichtung (14) und ein Verbindungselement (18; 18"'), welches Verbindungselement (18; 18"') einen ersten Befestigungsabschnitt (20) zum Festlegen an einer ersten Knochenbefestigungseinrichtung (12), einen zweiten Befestigungsabschnitt (22) zum Festlegen an einer zweiten Knochenbefestigungseinrichtung (14) und einen zwischen dem ersten und dem zweiten Befestigungsabschnitt (20, 22) angeordneten oder ausgebildeten, mindestens teilweise flexiblen Zwischenabschnitt (24; 24"') umfasst, welcher Zwischenabschnitt (24; 24"') mindestens eine Ausnehmung (66, 68) definiert, die derart ausgebildet ist, dass sie eine Verformung des Zwischenabschnitts (24; 24"') ermöglicht, **gekennzeichnet durch** eine chirurgische Vorrichtung (106) zum temporären Versteifen des flexiblen Zwischenabschnitts (24; 24"') des Verbindungselements (18; 18"') nach einem der voranstehenden Ansprüche.

15. Wirbelsäulenstabilisierungssystem nach Anspruch 14, **dadurch gekennzeichnet, dass** der Zwischenabschnitt (24) in Form eines streifenförmigen, gewundenen Blattfederelements (25) ausgebildet ist und mindestens eine in einer Richtung quer zu einer vom Zwischenabschnitt (24) definierten Längsachse (40) seitlich geöffnete Ausnehmung (66, 68) aufweist.

## Claims

1. Surgical device (106) for temporarily stiffening an at least partially flexible intermediate section (24; 24"') of a connecting element (18; 18"') for a spinal column stabilization system (10), said connecting element (18; 18"') comprising a first attachment section (20) for fixing to a first bone fixation device (12), a second attachment section (22) for fixing to a second bone fixation device (14), and said at least partially flexible intermediate section (24; 24"') arranged or formed between said first and second attachment sections (20, 22), said intermediate section (24; 24"') defining at least one recess (66, 68) which is configured so as to enable deformation of said intermediate section (24; 24"'), said surgical device (106) comprising at least one blocking element (108, 110) which is adapted to be brought into engagement with said intermediate section (24; 24"') at least partially with a positively locking connection for temporarily preventing deformation of said flexible intermediate section (24; 24"'), **characterized by** at least two retaining jaws (102, 104; 132; 142; 150; 162, 176; 182) which are movable in a direction of movement towards each other and away from each other and have retaining faces (112, 114; 138; 144; 154; 164; 178; 184) that face each other, at least one of the retaining jaws (102, 104) carrying or comprising the at least one blocking element (108, 110).

2. Surgical device in accordance with claim 1, **characterized in that** the at least two retaining jaws (102, 104) comprise a total of at least two axial stops (124, 126) that face each other and are configured so as to act relative to each other in a direction transverse to the direction of movement of the retaining jaws (102, 104) in order to prevent movement of the intermediate section (24) in the axial direction (40).

3. Surgical device in accordance with claim 1 or 2, **characterized by** a locking mechanism (93) for preventing movement of the retaining jaws (102, 104; 132; 142; 150; 162, 176; 182) in the direction of movement away from each other in a blocking position.

4. Surgical device in accordance with any one of the preceding claims, **characterized in that** the at least one blocking element (108) is configured in the form of a blocking projection (128) which protrudes from a retaining face (112) of one of the retaining jaws (102) or **in that** the at least one blocking element (108) is configured in the form of a blocking recess (116) provided in the retaining face (112) on one of the retaining jaws (102) for at least partially receiving the intermediate section (24) to be temporarily stiffened.

5. Surgical device in accordance with claim 4, **characterized in that** the blocking projection (128; 134) is of wedge-shaped or substantially wedge-shaped configuration.

6. Surgical device in accordance with claim 4, **characterized in that** at least one of the axial stops (124, 126) is formed on a blocking recess (116).

7. Surgical device in accordance with any one of the preceding claims, **characterized in that** the at least two retaining jaws (102, 104; 132; 142; 150; 162, 176; 182) differ in their configuration.

8. Surgical device in accordance with claim 7, **characterized in that** the retaining jaws (102, 104; 132; 142; 150; 162, 176; 182) comprise differently shaped blocking elements (108, 110).

9. Surgical device in accordance with any one of the preceding claims, **characterized by** a coupling device (190; 190') for releasably connecting the two retaining jaws (102, 104; 132; 142; 150; 162, 176; 182) to each other in a coupling position.

10. Surgical device in accordance with claim 9, **characterized in that** the coupling device (190; 190') comprises first and second coupling elements (192, 194; 192') which are arranged or formed, on the one hand, on the one retaining jaw (102, 104; 132; 142; 150; 162, 176; 182), and, on the other hand, on the other retaining jaw (102, 104; 132; 142; 150; 162, 176; 182) and are in engagement in the coupling position.

11. Surgical device in accordance with claim 9 or 10, **characterized in that** the coupling device (190') comprises a coupling bridge (192') or a thread.

12. Surgical device in accordance with any one of the preceding claims, **characterized by** an actuating device (81) for transferring the device (106) from an applying position in which the device (106) and the intermediate section (24; 24"') are disengaged to a blocking position in which the intermediate section (24; 24"') is held by the device (106) in an undeformable or substantially undeformable manner on the device (106).

13. Surgical device in accordance with claim 12, **characterized in that** the actuating device (81) comprises two actuating members (82, 84) movable relative to each other, which are directly or indirectly coupled to the at least two retaining jaws (102, 104; 132; 142; 150; 162, 176; 182) for moving these.

14. Spinal column stabilization system (10) comprising at least one first bone fixation device (12), at least one second bone fixation device (14) and a connecting element (18; 18"'), said connecting element (18; 18"') comprising a first attachment section (20) for fixing to a first bone fixation device (12), a second attachment section (22) for fixing to a second bone fixation device (14), and an at least partially flexible intermediate section (24; 24"') arranged or formed between the first and second attachment sections (20, 22), said intermediate section (24; 24"') defining at least one recess (66, 68) which is configured so as to enable deformation of said intermediate section (24; 24"'), **characterized by** a surgical device (106) for temporarily stiffening said flexible intermediate section (24; 24"') of said connecting element (18; 18"') in accordance with any one of the preceding claims.

15. Spinal column stabilization system in accordance with claim 14, **characterized in that** the intermediate section (24) is configured in the form of a strip-shaped, wound leaf spring element (25) and comprises at least one recess (66, 68) which is open at the side in a direction transverse to a longitudinal axis (40) defined by the intermediate section (24).

## Revendications

1. Appareillage chirurgical (106) pour rigidifier temporairement un tronçon intermédiaire (24; 24"') au moins partiellement flexible, d'un élément de liaison (18; 18"') pour un système de stabilisation de colonne vertébrale (10), ledit élément de liaison (18; 18"') comprenant un premier tronçon de fixation (20) destiné à être fixé à un premier dispositif de fixation à l'os (20), un deuxième tronçon de fixation (22) destiné à être fixé à un deuxième dispositif de fixation à l'os (14), et le tronçon intermédiaire (24; 24"') au moins partiellement flexible agencé ou formé entre le premier et le deuxième tronçon de fixation (20, 22), ledit tronçon intermédiaire (24; 24"') définissant au moins un évidement (66, 68) qui est configuré de manière à permettre une déformation du tronçon intermédiaire (24; 24"'), l'appareillage chirurgical (106) comprenant au moins un élément de blocage (108, 110) qui peut être amené, au moins partiellement par complémentarité de formes, en prise avec le tronçon intermédiaire (24; 24"') pour empêcher temporairement une déformation du tronçon intermédiaire flexible (24; 24"'), **caractérisé par** au moins deux mors de maintien (102, 104; 132; 142; 150; 162, 176; 182), qui peuvent être déplacés dans une direction de mouvement de manière à se rapprocher ou à s'éloigner l'un de l'autre, et qui comportent des surfaces de maintien (112, 114; 138; 144; 154; 164, 178; 184) dirigées l'une vers l'autre, au moins l'un des mors de maintien (102, 104) portant ou englobant ledit au moins un élément de blocage (108, 110).

2. Appareillage chirurgical selon la revendication 1, **caractérisé en ce que** lesdits au moins deux mors de maintien (102, 104) comprennent au total au moins deux butées axiales (124, 126) dirigées l'une vers l'autre, qui sont réalisées de manière à agir relativement l'une par rapport à l'autre dans une direction transversale à la direction de déplacement des mors de maintien (102, 104), en vue d'empêcher un mouvement de déplacement du tronçon intermédiaire (24) dans la direction axiale (40).

3. Appareillage chirurgical selon la revendication 1 ou la revendication 2, **caractérisé par** un dispositif de verrouillage (93) destiné à empêcher, dans une position de blocage, un mouvement de déplacement d'éloignement réciproque des mors de maintien (102, 104; 132; 142; 150; 162, 176; 182) dans la direction de mouvement.

4. Appareillage chirurgical selon l'une des revendications précédentes, **caractérisé en ce que** ledit au moins un élément de blocage (108) est réalisé sous la forme d'une protubérance de blocage (128) faisant saillie d'une surface de maintien (112) de l'un des mors de maintien (102), ou **en ce que** ledit au moins un élément de blocage (108) est réalisé sous la forme d'un évidement de blocage (116) prévu sur l'un des mors de maintien (102), dans la surface de maintien (112), pour accueillir au moins partiellement le tronçon intermédiaire (24) à rigidifier temporairement.

5. Appareillage chirurgical selon la revendication 4, **caractérisé en ce que** la protubérance de blocage (128; 134) est d'une configuration en forme de coin ou sensiblement en forme de coin.

6. Appareillage chirurgical selon la revendication 4, **caractérisé en ce que** l'une au moins des butées axiales (124, 126) est réalisée au niveau d'un évidement de blocage (116).

7. Appareillage chirurgical selon l'une des revendications précédentes, **caractérisé en ce que** lesdits au moins deux mors de maintien (102, 104; 132; 142; 150; 162, 176; 182) sont de configuration différente.

8. Appareillage chirurgical selon la revendication 7, **caractérisé en ce que** les mors de maintien (102, 104; 132; 142; 150; 162, 176; 182) comprennent des éléments de blocage (108, 110) formés différemment.

9. Appareillage chirurgical selon l'une des revendications précédentes, **caractérisé par** un dispositif de couplage (190; 190') pour relier de manière amovible l'un à l'autre, les deux mors de maintien (102, 104; 132; 142; 150; 162, 176; 182), dans une position de couplage.

10. Appareillage chirurgical selon la revendication 9, **caractérisé en ce que** le dispositif de couplage (190; 190') comprend des premiers et deuxièmes éléments de couplage (192, 194; 192'), qui sont agencés ou formés d'une part sur un mors de maintien (102, 104; 132; 142; 150; 162, 176; 182) et d'autre part sur l'autre mors de maintien (102, 104; 132; 142; 150; 162, 176; 182), et sont en prise dans la position de couplage.

11. Appareillage chirurgical selon la revendication 9 ou la revendication 10, **caractérisé en ce que** le dispositif de couplage (190') comprend une nervure de couplage (192') ou un fil.

12. Appareillage chirurgical selon l'une des revendications précédentes, **caractérisé par** un dispositif d'actionnement (81) pour transférer l'appareillage (106) d'une position de mise en place ou d'application, dans laquelle l'appareillage (106) et le tronçon intermédiaire (24; 24"') sont hors de prise réciproque, à une position de blocage dans laquelle, à l'aide de l'appareillage (106), le tronçon intermédiaire (24; 24"') est maintenu de manière indéformable ou sensiblement indéformable sur l'appareillage (106).

13. Appareillage chirurgical selon la revendication 12, **caractérisé en ce que** le dispositif d'actionnement (81) comprend deux organes d'actionnement (82, 84) mobiles l'un par rapport à l'autre, qui sont couplés directement ou indirectement avec lesdits au moins deux mors de maintien (102, 104; 132; 142; 150; 162, 176; 182) pour produire leur mouvement de déplacement.

14. Système de stabilisation de colonne vertébrale (10) comprenant au moins un premier dispositif de fixation à l'os (12), au moins un deuxième dispositif de fixation à l'os (14), et un élément de liaison (18; 18"'), ledit élément de liaison (18; 18"') comprenant un premier tronçon de fixation (20) destiné à être fixé à un premier dispositif de fixation à l'os (20), un deuxième tronçon de fixation (22) destiné à être fixé à un deuxième dispositif de fixation à l'os (14), et un tronçon intermédiaire (24; 24"') au moins partiellement flexible, agencé ou formé entre le premier et le deuxième tronçon de fixation (20, 22), ledit tronçon intermédiaire (24; 24"') définissant au moins un évidement (66, 68) qui est configuré de manière à permettre une déformation du tronçon intermédiaire (24; 24"'), **caractérisé par** un appareillage chirurgical (106) selon l'une des revendications précédentes, pour rigidifier temporairement le tronçon intermédiaire flexible (24; 24"') de l'élément de liaison (18; 18''').

15. Système de stabilisation de colonne vertébrale selon la revendication 14, **caractérisé en ce que** le tronçon intermédiaire (24) est réalisé en tant qu'élément en lame de ressort (25), sinueux, en forme de bande, et présente au moins un évidement (66, 68) ouvert latéralement dans une direction transversale à un axe longitudinal (40) défini par le tronçon intermédiaire (24).
